# EUROPEAN PATENT APPLICATION

(11) **EP 2 366 788 A1**
(43) Date of publication of application: **21.09.2011**
(21) Application number: 10169875.1
(22) Date of filing: 26.07.2002
(51) Int. Cl.: C12N 15/82, C12N 15/74, C12N 5/10, A01H 5/00, C12N 9/10, C12N 15/11

(54) **Transformation method for obtaining marker-free plants**

(30) Priority: 27.07.2001 EP 01202878
(62) Divisional of application: 02747761.1
(71) Applicant: Coöperatie Avebe U.A., 9607 PT Foxhol (NL)
(72) Inventor: Wolters, Anna Maria Agnes, 6708 RE Wageningen (NL); Visser, Richard Gerardus Franciscus, 6721 ET Bennekom (NL); van der Meer, Ingrid Maria, 6703 AV Wageningen (NL); Heeres, Paul, 7876 CG Valthermond (NL); de Vetten, Nicolaas Clemens Maria Henricus, 9731 KH Groningen (NL)
(74) Representative: Jansen, Cornelis Marinus

(57) **Abstract**

The invention relates to a transformation method for obtaining transgenic plants and plants obtained with said method. The invention provides a method for transforming a plant cell comprising providing a plant cell with a recombinant nucleic acid comprising a T-DNA construct allowing for transfer of said construct into the genome of a plant cell, said construct provided with a foreign nucleic acid that is free of nucleic acid encoding a selective marker.

## Description

Transformation of plants using transforming bacteria such as Agrobacterium spp. to obtain transgenic plants expressing a heterologous gene or gene fragment of interest has been known since the late seventies and early eighties of the last century when it was found that the Ti (tumor inducing) plasmid of said bacterium could be used as a vector in genetic engineering of plants. The wild-type plasmid induces plant cells to produce tumor cells, but it can be modified so that it can carry foreign gene constructs into cells without necessarily making the recipient cells tumorous. During tumor induction, a specific segment of the Ti plasmid, called the T-DNA (transferred DNA), integrates into the host plant nuclear DNA. In genetic engineering of plants, said T-DNA is modified and now carries the foreign gene construct to be integrated in the plant's DNA so that a transgenic plant can be obtained.

A transformation procedure for obtaining transgenic plants generally consists of infection of a plant cell with a transforming bacterium, which generally comprises an essentially non-tumorigenic Agrobacterium strain, which bacterium is provided with a recombinant nucleic acid comprising a T-DNA vector construct allowing for transfer of said construct into the genome of a plant cell, said construct essentially comprising the desired nucleic acid, gene or gene fragment that one wishes to see expressed in a finally transformed plant and a selective marker nucleic acid or selection gene. This desired heterologous gene and the marker are in general located on a plasmid or vector in a piece of the T-DNA, which is the DNA flanked by at least one, or located between two imperfect direct repeats of most often 24 basepairs length, called the T-DNA borders. Transfer of the heterologous gene/selection gene construct into the plant cell takes place in a process whereby vir genes (located on the same or different plasmid) are involved to accommodate transfer and integration of the T-DNA/gene construct. Vir-proteins (D1 and D2) cause nicking of the border repeats at a precise site whereby the T-DNA construct is cut at the T-DNA borders from the plasmid and inserted into the plant genome.

By growing the thus treated plant cells in an appropriate environment, whole plants can be regenerated, some of which carry the desired gene. To select for the desired transformed cells in the background of untransformed cells DNA encoding said selection gene and means for its expression is generally physically linked to the gene of interest on the T-DNA to permit the recovery of transformants. The presence of such a selection or marker sequence in the T-DNA is generally seen as an absolute requirement for efficient recovery; otherwise tenths- to hundreds-of-thousands putative transformants would need to be screened for the presence and functional insertion of the desired heterologous gene. Instead, putative transformants are always grown in a selective medium or under selective pressure appropriate for the selective marker chosen to give the transformed cells a selective advantage over the, initially over-abundantly present, non-transformed cells.

Historically, a plant selection marker is a dominant gene which, after expression, confers resistance to a selective agent that is added to the regeneration medium, but which itself is not essential for cell growth. Such a selective agent is for example an antibiotic, herbicide, amino acid or amino acid analog added to a plant or plant culturing medium in a toxic concentration. Among the selective markers or selection genes that are most widely used in plant transformation are the bacterial neomycin phosphotransferase genes (nptI, nptII and nptIII genes) conferring resistance to the selective agent kanamycin, suggested in EP 131623 and the bacterial aphIV gene suggested in EP 186425 conferring resistance to hygromycin. EP 275957 discloses the use of an acetyl transferase gene from *Streptomyces viridochromogenes* that confers resistance to the herbicide phosphinotricin. Plant genes conferring relative resistance to the herbicide glyphosate are suggested in EP218571. The resistance is based on the expression of a gene encoding 5-enolshikimate-3-phosphate synthase (EPSPS) that is relatively tolerant to N-phosphomethylglycine. Certain amino acids such as lysine, threonine, or the lysine derivative amino ethyl cysteine (AEC) and tryptophan analogs like 5-methyl tryptophan can also be used as selective agents due to their ability to inhibit cell growth when applied at high concentration. In this selection system expression of the selectable marker gene results in overproduction of amino acids by transgenic cells which permits the transgenic to grow under selection.

Another class of selectable markers are those that support growth and proliferation of the transformed plant cells under conditions that are insufficient for the growth of non-transformed cells, e.g. conditions lacking a plant growth hormone. A selection marker gene can encode an enzyme that after expression converts an encryptic carbon source into a carbon source that supports growth and proliferation of the transformed plant cells under conditions that contains minimal nutrients and in which the carbon source is replaced by an encryptic or latent carbon source that can not be utilized by non-transformed cells. An example of such a selection marker is the phosphomannose isomerase that converts non-utilizable mannose-6-phosphate into fructose-6-phosphate that can be used by plant cells as a carbon source (suggested in US6143562) or xylose isomerase from *Streptomyces rubiginosus* (Haldrup A., et al. 1998, Plant Cell Report 18:76-81).

Yet another class of selection marker genes allows the screening of presumably transformed plant cells rather than direct genetic selection of transformed cells for resistance to a toxic substance such as an antibiotic. For this reason they are often also called screenable markers. The genes are referred to as reporter genes and include for instance beta-glucuronidase (GUS), beta-galactosidase, luciferase, chloramphenicol acetyltransferase and green fluorescent protein (GFP).

Alternatively, a screenable marker may provide some other visible reactive response. For instance, it may cause a distinctive appearance or growth pattern relative to plants or plant cells not expressing a screenable marker gene in the presence of a substance, which can either be applied to the plant or plant cells directly or a substance which is present in the plant or in the plant cell growth media.

Generally, the plants or plant cells containing such screenable marker genes have a distinctive phenotype for purpose of identification, i.e., they can be distinguished from non-transformed cells. The characteristic phenotype allows the identification of cells, cell groups, tissues, organs, plant parts or whole plants containing the construct. An example of a morphological abnormality induction (MAI) marker gene is the isopentenyl transferase gene from Agrobacterium (Keller et al. WO 00/37060). Isopentenyl transferase is a rate-limiting enzyme in the biosynthesis of cytokinin, which is a plant growth hormone. A plant cell into which the *ipt* gene is introduced produces cytokinin, with the result that the proliferation and differentiation of the cell containing the *ipt* gene are confused to induce various morphological abnormalities.

The presence of antibiotic resistance genes and other selective markers sequences in the final transgenic plant obtained is in most cases considered undesirable. These sequences, albeit thought to be necessary for the transformation processes, do in general not positively contribute to the final transformed plant and in fact lessen its desirability to the consumer for a number of reasons. Consumer groups express concern about the widespread distribution of resistance markers in food products referring to a theoretical risk of a horizontal transfer of transgene selection genes into gut bacteria.

Environmental groups are concerned about the risk of cross-pollination between the transgenic plant and related species which can lead to a transfer of resistance traits into weeds, jeopardizing the long-term use of transgenic crops and causing potential ecological problems. Consequently, generating marker-free plants would likely alleviate the public weariness towards acceptance of transgenic crops.

A number of systems have so far been developed to facilitate the removal of selective marker genes. Co-transformation of two different constructs can result in transgenic lines that have integrated both transgenes and after a genetic cross the selective marker can be segregated away from the gene of interest. Removal of selective marker genes by co-transformation is suggested in e.g. WO95/16031, US 6265638 and WO00/18939. A co-transformation system however requires that the marker gene is located at an unlinked location meaning screening of many more independent transformation events. Moreover, many cross pollinating and in particular vegetatively propagated crops, and especially tuberous crops like potato and cassava, are highly heterozygous and removal of the marker sequence by genetic segregation would require many years to find a clone with suitable field performance. Several transposable element systems and site-specific recombination systems have been employed for marker removal (Sugita K, et al. 2000 Plant J 22: 461-469; Zuo J, et al. 2001 Nature Biotech 19: 157-161). EP 716147 describes a vector for introducing a desired gene into plants, comprising the gene of interest and at least a morphological abnormality induction (MAI) marker gene placed on a removable DNA element i.e. a transposable element or site-specific recombination system. Plants transformed with the MAI containing gene construct can be easily detected by eye by their abnormal morphology of the shoots. Likewise, the loss of the MAI gene's function after transposition of the transposable element or after site-specific removal of the recombination system can be easily detected as this results in normal looking shoots. Such transgenic plants can be produced free of marker genes without having to undergo the crossing step. These systems require the expression of a transposase or recombinase that mediates the deletion of regions bracketed between recombination or transposase target sequences, and the subsequent removal of the marker gene by genetic segregation. Also these systems are time consuming as well as impractical for species that are mainly propagated vegetatively. Moreover, deleted fragments can reinsert into other genomic positions. Another approach to induce DNA deletions is based on intrachromosomal homologous recombination between two homologous sequences. Intrachromosomal homologous recombination frequencies are often too low for an efficient application of this system to produce deletions of transgene regions. Using the attP region of bacteriophage λ this recombination frequency could be increased (Zubko E, et al. 2000 Nature Biotech 18: 442-445). However, the process is still unpredictable and too inefficient to generate hundreds to thousands of independent transformation events to find the clone with suitable agronomical performance. A system to recover transgenic cells without the use of selective markers has been described. In WO98/51806 a method is disclosed for the recovery of transformed cells without the use of selectable markers by enrichment of transgenic sectors using nodal culture and non-selective screening assays. This method involves the culturing of the transformed plant cells or tissue comprising a non-selectively assayable transgene until nodes comprising meristematic tissue have developed. Subsequently, the plant tissue is assayed using a non-selective assay, such as enzyme assays or ELISA's. Assay-positive plants that are recovered with this method are chimeric and have transformed sectors. To enrich these transformed sector from the assay-positive tissue nodal explants are prepared and cultured such that shoots are formed. Shoots and leaves are to be assayed again using a non-selective assay in the hope that plants are recovered with enriched transformed sectors so that eventually, after several rounds of assaying, a near-uniform transgenic plants can be obtained.

The invention provides a method for producing transgenic plants, in particular of cross pollinated and vegetatively propagated crops, that contain a gene of interest and that are essentially free of other foreign or heterologous ancillary nucleic acids such as selective marker genes and which thus do not need to be selected through treatment with a selective agent. All of the above mentioned systems for the generation of marker-free transformants are employed on the assumption that isolation of non-chimeric transformants without a selective marker is practically unfeasible, at least with transformation protocols using T-DNA constructs or corresponding transforming bacteria. The method provided herein allows for the production of plants which contain a desired gene, but which are essentially free of vector sequences and/or marker sequences used to transform the plant. Surprisingly, the invention provides a method for the transformation of a plant or plant cell comprising using a transforming bacterium, such as Agrobacterium spp., and its T-DNA to obtain non-chimeric transgenic plants expressing a desired heterologous gene of interest, wherein said T-DNA is provided with the desired gene or gene fragment but does not comprise an additional selection gene or fragment thereof.

For this purpose, the invention provides an isolated or recombinant nucleic acid comprising a T-DNA or functional equivalent thereof allowing for transfer of said T-DNA into the genome or nuclear DNA of a plant cell, said T-DNA provided with a nucleic acid that is free of a nucleic acid encoding a selective marker, and the use of such a T-DNA construct in genetic engineering of plants. Consequently, the method provided herein does not require growing putative transformants in selective medium or under selective pressure to let the truly transformed plant emerge. Instead, selection for the desired transformant can easily be achieved by testing for the presence of the desired heterologous gene or gene fragment or construct itself, for example by using commonly known nucleic acid techniques, such as detection by Polymerase Chain Reaction or by hybridisation with complementary sequences in routine Southern blotting experiments. It will also be apparent to those skilled in the art that the presence of a desired heterologous gene or gene fragment can be assayed by monitoring the presence or the absence or change in amount of the expression product of the gene. For example, an expressed protein allowing detection by ELISA (enzyme-linked immunosorbent assay) the presence of such a protein can be assayed by ELISA. Furthermore, the method provided herein may involve a bioassay or a chemical analytical method such as gass chromatography/mass spectometry (GC/MS). Testing need to be done for the presence or absence of the recombinant T-DNA construct used for transfer of said construct into the DNA of the plant cell, especially for that part of the construct provided with the desired foreign nucleic acid that is free of nucleic acid encoding a selective marker. It is preferred that a nucleic acid according to the invention comprises a T-DNA construct which comprises at least one T-DNA border, but for ease of integration preferably said foreign nucleic acid is flanked by T-DNA border repeats or functional equivalents thereof. The VirD1 and VirD2 proteins of the Agrobacterium host cell recognize the border repeat sequences and produce a single stranded nick between the third and the fourth base in the bottom strand of each border repeat. These nicks determine the initiation termination sites of the T-strand at the right and left border, respectively. The left border was found to be non-essential for transfer of the T-DNA, but helps to define the left end of the T-DNA. The right border seems to be most essential in T-DNA transfer. Ti plasmids with the T-DNA right border region deleted are in general avirulent. (Holsters, M. et al. Plasmid 3, 212-230, 1980). Deletion of the left border region has no effect on virulence. The sequence context of the repeats determine their relative activity during T-DNA transfer (Wang et al. Mol. Gen. Genet. 210, 338-346, 1980). A typical example of a border repeats of the pBIN19 plasmid is Right Border 5'-TGACAGGATATATTGGCGGGTAAAC-3' and Left Border 5'-TGGCAGGATATATTGTGGTGTAAAC-3'. Of course, practicing the invention is most easily achieved with a nucleic acid according to the invention wherein said T-DNA is derived from the Ti plasmid of an Agrobacterium spp, especially wherein said Agrobacterium comprises *A. tumefaciens,* said bacterium and expertise being most widely available.

In one embodiment, a nucleic acid according to the invention is provided wherein said foreign nucleic acid allows for regulation of the expression of a target gene in said genome. Both upregulation (or overexpression) and downregulation can be achieved by "sense" technology. If a full length copy of the target gene is inserted into the genome a range of phenotypes can be obtained, some overexpressing the target gene, some under-expressing. A population of plants produced by this method may then be screened and individual phenotypes isolated. A preferred embodiment comprises a method wherein said regulation comprises downregulation. The inhibition of expression of a target gene, commonly referred to as "gene silencing" can be achieved by "antisense downregulation" and "sense downregulation" (also, referred to as "cosuppression"). In antisense downregulation, a DNA fragment which is complementary to all or part of an endogenous target gene is inserted into the genome in reverse orientation. While the mechanism has not been fully elucidated, one theory is that transcription of such an antisense gene produces mRNA which is complementary in sequence to the mRNA product transcribed from the endogenous gene. The antisense mRNA then binds with the naturally produced "sense" mRNA to form a duplex which inhibits translation of the natural mRNA to protein. Antisense downregulation technology is well-established in the art and used routinely in laboratories around the world. Gene silencing can therefore be achieved by inserting into the genome of a target organism a copy of at least a fragment of the target gene coding sequence which copy may comprise either the whole or part or be a truncated sequence and may be in sense or antisense orientation. Additionally, intron sequences which can be obtained from the genomic gene sequence may be used in the construction of suppression vectors. There have also been reports of gene silencing being achieved within organisms of both the transgene and the endogenous gene where the only sequence identity is within the promoter regions.

In a much preferred embodiment, the invention provides a T-DNA construct wherein said foreign nucleic acid includes an inverted repeat of at least part of a polynucleotide region of said target gene. Although antisense and sense downregulation can result in complete silencing of the target gene, the efficiency is generally not very high. A maximum of 25% of the antisense transformants displayed complete silencing, while only about 10% of the transformants obtained with sense constructs showed some level of silencing (Smith et al. 2000 Nature 407: 319-320; Wolters and Visser, 2000 Plant Mol Biol 43: 377-386). Recently, it has been observed that the inhibition of a selected target gene within an organism is enhanced, when the gene silencing vector includes a inverted repeat of all or part of a polynucleotide region of the target gene.

The inverted repeat sequence may consist of a T-DNA with one promoter driving expression of a sense copy of the cDNA together with another promoter in front of an antisense copy of the same cDNA (Chuang and Meyerowitz, 2000 Proc Natl Acad Sci USA 97: 4985-4990) or the T-DNA may contain a cDNA sequence flanked on both ends by a promoter (LaCount et al. 2000 Mol Biochem Parasit 111: 67-76), or the T-DNA may contain a promoter driving transcription of an inverted repeat of (part of) the cDNA (Hamilton et al. 1998; Smith et al. 2000 Nature 407: 319-320; Wang and Waterhouse, 2000 Wang MB, Waterhouse PM (2000) Plant Mol Biol 43: 67-82) or the promoter (Mette et al. 2000 EMBO J 19: 5194-5201) of the gene to be silenced. Some inverted repeat constructs are reported to result in 100% of the transformants displaying silencing of the target gene (Smith et al. 2000 Nature 407: 319-320) in case of using an intron as a spacer between the repeats. The spacer fragment contributes to the stability of the perfect inverted repeat sequences, but is not required for the specificity of the silencing.

In a particularly preferred embodiment of a nucleic acid according to the invention said target gene encodes a granule-bound starch synthase (GBSSI) enzyme. The detailed description herein gives a striking increase in the frequency of completely silenced transformants following the inclusion of a short repeated region of the target gene encoding the granule-bound starch synthase (GBSSI) enzyme of potato within a transgene.

Also is provided a nucleic acid according to the invention wherein said foreign nucleic acid allows for expression of a heterologous polypeptide in said plant cell. Suitable polypeptides are manifold, typical examples of foreign nucleic acid or genes that are of interest to transfer marker-free into plants are those encoding for proteins and enzymes that modify metabolic and catabolic processes. Other examples are genes that may encode a protein giving added nutritional value to the plant as a food or crop. Typical examples include plant proteins that can inhibit the formation of anti-nutritive factors and plant proteins that have a more desirable amino acid composition (e.g. a higher lysine content than the non-transgenic plant). In a preferred embodiment, said nucleic acid or gene of interest encodes a polypeptide which comprises an enzyme. The gene of interest may code for an enzyme that can be used in food processing such as chymosin, thaumatin and alpha-galactosidase. The gene of interest may also code for an agent for introducing or increasing pathogen resistance. The gene of interest may code for a non-natural plant compound that is of benefit to animals or humans. For example, the gene of interest could code for a pharmaceutically active protein or enzyme such as any one of the therapeutic compounds insulin, interferon, human serum albumin, human growth factor and blood clotting factors. In this regard, the transformed cell or organism could prepare acceptable quantities of the desired compound which would be easily retrievable from, for example, the tubers. Preferably, the gene of interest is a gene encoding for a protein or peptide having a high nutritional value, a feedback-insensitive amino acid biosynthesis enzyme such as dihydrodipicolinate synthase (EC 4.2.1.52, DHPS), an enzyme or peptide conferring disease resistance, a sense or antisense transcript such as that for patatin, ADP-glucose pyrophosphorylase, alpha-amylase, branching enzyme, granule-bound starch synthase, soluble starch synthases, a protease or a glucanase.

The invention also provides a vector or plasmid comprising a nucleic acid according to the invention, and host cells comprising such vectors or nucleic acid. A preferred host cell comprises an Agrobacterium. For practicing the method of transformation according to the invention it is even preferred to use a substantially virulent Agrobacterium such as *A*. *tumefaciens,* as exemplified by strain A281 or a strain derived thereof or another virulent strain available in the art. These Agrobacterium strains carry a DNA region originating from the virulence region of the Ti plasmid pTiBo542 containing the *virB, virC* and *virG* gees. The virulence (vir) gene products of *A*. *tumefaciens* coordinate the processing of the T-DNA and its transfer into plant cells. Vir gene expression is controlled by *virA* and *virG,* whereby *virA* upon perception of an inducing signals activates *virG* by phosphorylation. *VirG*, in turn, induces the expression of *virB,C,D,E*. These genes code for proteins involved in the transfer of DNA. The enhanced virulence of pTiBo542 is thought to be caused by a hypervirulent *virG* gene on this Ti plasmid (Chen et al. Mol. Gen. Genet 230: 302-309, 1991). However, besides by Agrobacterium infectium, there are other means to effectively deliver of DNA to recipient plant cells when one wishes to practice the invention. Suitable methods for delivering DNA to plant cells are believed to include virtually any method by which DNA can be introduced into a cell, such as by direct delivery of DNA such as by PEG-mediated transformation of protoplasts (Omirulleh et al., 1993), by desiccation/inhibition-mediated DNA uptake (Potrykus et al., Mol. Gen. Genet., 199:183-188, 1985), by electroporation (U.S. Pat. No. 5,384,253), by agitation with silicon carbide fibers (Kaeppler et al., 1990; U.S. Pat. No. 5,302,523; and U.S. Pat. No. 5,464,765), and by acceleration of DNA coated particles (U.S. Pat. No. 5,550,318; U.S. Pat. No. 5,538,877; and U.S. Pat. No. 5,538,880). Through the application of techniques such as these, certain cells from virtually any plant species may be stably transformed, and these cells developed into transgenic plants.

In Microprojectile Bombardment, particles may be coated with nucleic acids and delivered into cells by a propelling force. Exemplary particles include those comprised of tungsten, gold, platinum, and the like. It is contemplated that in some instances DNA precipitation onto metal particles would not be necessary for DNA delivery to a recipient cell using microprojectile bombardment. However, it is contemplated that particles may contain DNA rather than be coated with DNA. For microprojectile bombardment transformation in accordance with the current invention, both physical and biological parameters may be optimized. Physical factors are those that involve manipulating the DNA/microprojectile precipitate or those that affect the flight and velocity of either the macro- or microprojectiles. Biological factors include all steps involved in manipulation of cells before and immediately after bombardment, such as the osmotic adjustment of target cells to help alleviate the trauma associated with bombardment, the orientation of an target tissue relative to the particle trajectory, and also the nature of the transforming DNA, such as linearized DNA or intact supercoiled plasmids. It is believed that pre-bombardment manipulations are especially important for successful transformation.

Accordingly, it is contemplated that one may wish to adjust various bombardment parameters in small scale studies to fully optimize the conditions. One may particularly wish to adjust physical parameters such as DNA concentration, gap distance, flight distance, tissue distance, and helium pressure. It is further contemplated that the grade of helium may effect transformation efficiency. One also may optimize the trauma reduction factors by modifying conditions which influence the physiological state of the recipient cells and which may therefore influence transformation and integration efficiencies. For example, the osmotic state, tissue hydration and the subculture stage or cell cycle of the recipient cells may be adjusted for optimum transformation.

The transforming DNA used for microprojectile transformation may contain an expression cassette comprising generally of a cDNA, gene or genes which one desires to introduce into the cells, and still further, may include a promoter and 3' region operatively linked to the exogenous gene. The optimized gene construct is described in present invention. The DNA segment may additionally include a second, third, fourth, fifth, sixth, or any additional number of exogenous genes capable of being placed on a single DNA molecule and transformed into a recipient cell.

In particular embodiments of the invention, the DNA segments will not include a transformation selectable or screenable marker gene. Generally one may employ a selectable or screenable marker gene in addition to, the expressible gene of interest in order to improve the ability to identify transformants. "Marker genes" are genes that impart a distinct phenotype to cells expressing the marker gene and thus allow such transformed cells to be distinguished from cells that do not have the marker. Such genes may encode either a selectable or screenable marker, depending on whether the marker confers a trait which one can "select" for by chemical means, i.e., through the use of a selective agent (e.g., a herbicide, antibiotic, amino acid analog or the like), or whether it is simply a trait that one can identify through observation or testing, i.e., by "screening" (e.g., the R-locus trait, beta-glucuronidase or uidA gene). For production of transformants free of vector DNA sequences it will be desirable to deliver DNA to cells that does not contain DNA sequences necessary for maintenance of the plasmid vector in the bacterial host, e.g., *E*. *coli,* such as antibiotic resistance genes, including but not limited to ampicillin, kanamycin, and tetracycline resistance, and prokaryotic origins of DNA replication. In such case, a DNA fragment containing the transforming DNA may be purified prior to transformation.. Purification can be achieved by for example gel electrophoresis on a agarose gel, followed by recovery of a DNA fragment from the agarose gel.

Control of the copy number of introduced transgenes and an efficiently production of low-copy transformants can be achieved by end-modification of nucleic acid segments by dephosphorylation or by blunting the ends of the nucleic acid segments (WO99/32642). The recipient plant cells for transformation with the microprojectile bombardment compositions of the invention may be from potentially any transformable monocot or dicot plant. Preferred monocot plant cells for use with the invention are from rice, wheat, barley, oats, rye, millet, sorghum, sugarcane, turfgrass and maize. Preferred dicot plant cells for use with the invention include cotton, tomato, citrus, tobacco, soybean and particularly potato and cassava.

After effecting delivery of exogenous DNA to recipient cells, the next steps generally concern identifying the transformed cells for further culturing and plant regeneration. In the present invention cells after delivery of exogenous DNA will be cultured in media that supports regeneration of plants. After shoot development DNA or RNA detection methods are used for detecting plantlet containing one or more transgenes. The method includes the isolation of nucleic acid from plantlets or a pool of plantlets according to standard methodologies (Sambrook et al., 1989). The invention provides the use of a nucleic acid detection method for determining whether a transformed plant cell or progeny thereof is transformed with a recombinant nucleic acid comprising testing said cell or said progeny for the presence or absence of said nucleic acid or gene product derived thereof. The nucleic acid may be genomic DNA, RNA or mRNA. For example, such a method can be used to detect both the presence of particular genes as well as rearrangements that may have occurred in the gene construct, i.e. in a kind of quality control to check whether a plant cell has been sufficiently transformed. The invention provides the use of a mRNA detection method for determining whether a nucleic acid construct in a transformed plant cell or progeny thereof is sufficiently integrated into a plant genome to be transcribed into a mRNA construct. The specific nucleic acid of interest, being part of the transgene is identified in the sample directly (DNA) or indirectly (RNA) using amplification. Next, the identified product is detected. In certain applications, the detection may be performed by visual means (e.g., ethidium bromide staining of a gel). Alternatively, the detection may involve indirect identification of the product via chemiluminescence, radioactive scintigraphy of radiolabel or fluorescent label or even via a system using electrical or thermal impulse signals. A variety of different assays are contemplated in the screening of transgenic plants created using the methods of the current invention. These techniques can be used to detect for both the presence of particular genes as well as rearrangements that may have occurred in the gene construct. The techniques include but are not limited to, polymerase chain reaction (PCR), fluorescent in situ hybridization (FISH), direct DNA sequencing, PFGE analysis, Southern or Northern blotting, single-stranded conformation analysis (SSCA), RNAse protection assay, allele-specific oligonucleotide (ASO), dot blot analysis, denaturing gradient gel electrophoresis, restriction fragment length polymorphism (RFLP) and PCR-SSCP or chip-based DNA technologies. The invention provides the use of a nucleic acid detection method for determining whether a transformed plant cell or progeny thereof is transformed with a recombinant nucleic acid comprising a T-DNA construct or a functionally equivalent nucleic acid construct allowing integration into a genome of a plant cell. Furthermore, the invention provides the use of a nucleic acid detection method for determining whether a transformed plant cell or progeny thereof is transformed with a recombinant nucleic acid comprising testing said cell or said progeny for the presence or absence of undesired vector material such as vector backbone sequences. For example, a method according to the invention can be used to check whether a transformed plant cell is essentially free of ancillary unwanted nucleic acids. The transformed plantlet, identified by nucleic acid detection methods, will then be allowed to mature into plants. After the regenerating plants have reached the stage of shoot and root development, they may be transferred to a greenhouse for further growth and testing.

Furthermore, the invention provides a plant cell comprising a nucleic acid according to the invention, and a regenerated plant, or part thereof, derived from such a plant cell. Particularly provided are tuberous plants, or parts thereof, which are preferably selected from the group of potato or cassave plants. Such a potato or cassave plant as provided herein comprises a so-called marker-free high-amylopectine tuber (for example potato or cassava), being essentially devoid of amylose due to downregulation of the gene encoding the granule-bound starch synthase enzyme. Such a potato or cassava plant contains an amylose content of less than 5%, and does essentially not contain any transformation selection marker genes like antibiotic or herbicide resistance genes. Further, preferably these plants have not integrated in their genome vector sequences used for the transformation process, like the whole or parts of Agrobacterium Ti plasmid, and contain low copy nummer of the T-DNA insert, preferably one copy. Also provided is a high lysine plant (preferably potato or cassava) that contains more than 20% lysine of the total free amino acid content, or more than 15% methionine of the total free amino acid content, or more than 30% amylose or more than 2% coagulated protein while essentially not containing transformation selection marker genes like antibiotic or herbicide resistance genes, not having integrated in their genome vector sequences used for the transformation process, like the entire or parts of Agrobacterium Ti plasmid, and containing low copy numbers of the T-DNA insert, preferably one copy.

The invention provides a method for transforming a plant cell comprising providing a plant cell with a recombinant nucleic acid according to the invention, comprising testing said cell or at least part of the progeny of said cell for the presence or absence of said nucleic acid or gene product derived thereof wherein said testing comprises the use of a nucleic acid detection method. In one embodiment, a nucleic acid detection method can be used to screen a population of putative transformants for the presence or the absence of a foreign nucleic acid in a plant cell of progeny derived thereof to identify the desired transformed cells in the background of untransformed cells. Particularly, a nucleic acid detection method, such as a PCR method, may be used to identify transformed tuberous plant cells, which are preferably selected from the group of potato or cassava plants. In one embodiment, the invention provides a method for the production and isolation of transformants showing silencing of an endogenous gene or overexpression of an endogenous gene, without the involvement of a selective marker gene.

We have shown herein that with this method it is even possible to obtain essentially marker gene-free, backbone vector DNA-free transformants, having advantageously only 1 insert of the T-DNA inserted, in which an endogenous gene is silenced or overexpressed. Further, it has been demonstrated that this method yields transformants which are uniform and non-chimeric. In another embodiment, a method is provided for the production and isolation of transformants wherein a heterologous gene is expressed, said transformants likewise being essentially marker-free and backbone vector DNA-free. Transformed cells that are identified by a method according to the invention can be cultured further and essentially marker-free plants can be obtained which carry the desired gene. The invention provides the use of a protein detection method for determining whether a recombinant nucleic acid construct in a transformed plant cell or progeny thereof is sufficiently integrated into a plant genome to allow for regulation of the expression of a target gene in the genome of a plant cell. A protein detection method according to the invention can also be used to determine the expression level of a protein in plant cells wherein the expression of a gene is regulated, for example when expression of a target gene is inhibited by using a gene silencing or downregulation technology. An enzyme assay or an immunoassay, for example an ELISA-type assay, Western blot, dot-blot analysis or the like, may be used to determine the extent of downregulation of a target gene or the level of overexpression of a gene following transformation of a plant cell with a foreign nucleic acid, allowing the selection of transformants. A protein detection method according to the invention allows to examine if a foreign nucleic is expressed in a plant cell or plant tissue during a certain developmental stage of a plant cell or progeny thereof. Such a foreign nucleic acid may encode a heterologous polypeptide, e.g. an enzyme. As said, a method is provided wherein it is not necessary to culture the truly transformed cell under selective pressure derived from a selective medium to outcompete untransformed cells, the efficiency of transformation using a method according to the invention is so high that such selective culturing is no longer required. The invention provides a method for transforming a plant cell comprising providing a plant cell with a recombinant nucleic acid comprising a T-DNA construct or a functional equivalent thereof wherein said plant cell is a tuberous plant cell, comprising testing said tuberous plant cell or at least part of the progeny thereof for the presence or absence of said nucleic acid or gene product thereof wherein said testing comprises the use of a nucleic acid detection method. A method according to the invention method is especially suitable to obtain a marker-free tuberous plant since tuberous plant are highly heterozygous and do essentially not allow outcrossing of a marker gene by conventional genetic segregation. All that is required in the present invention is testing at least a part of the progeny of said cell for the presence or absence of at least a functional part of a nucleic acid according to the invention. Such progeny can consist of parts of roots or shoots, or of individual cells, thereby leaving sufficient transformed material for further regeneration and vegetative propagation. It is preferred to select transformants such as transformed shoots through the use of a nucleic acid detection method and / or phenotypic screening. Transformants that carry the gene of interest and are free of ancillary nucleic acids may also be detected by Southern hybridization, Polymerase Chain Reaction procedures and other detection methods available in the art. Such testing may further comprise testing at least a part of the progeny of said cell for the presence or absence of undesired vector nucleic acid. In short, this invention provides methods for producing transgenic plants that contain a gene of interest and that are essentially free of ancillary unwanted nucleic acids. In a preferred embodiment the invention provides for transforming plants using an optimized transformation procedure using an (preferably virulent) Agrobacterium strain, an optimized gene construct for efficient inhibition or overexpression of a gene and an efficient method to detect transformants biochemically instead of due to selective pressure, for example, by Southern blot hybridization, Polymerase Chain Reaction (PCR) procedures or other nucleic acid detection methods available.

In another preferred embodiment, the invention provides a method for transforming a tuberous plant cell, providing said cell with a recombinant nucleic acid comprising a T-DNA construct that is free of nucleic acid encoding a selective marker, said construct provided with a target gene that encodes a granule-bound starch synthase (GBBI) enzyme, comprising testing at least part of the progeny of said tuberous plant cell for the presence or absence of said target gene wherein said testing comprises the use of a nucleic acid detection method, for example the use of PCR technology.

### Legends

- Figure 1.: Map of the GBSS cDNA antisense gene silencing vector
- Figure 2: Map of the plasmid pMTL1.1 containing the 1.1 kb 5' end of the GBSS cDNA used in the preparation of the plasmids of Figure 4, 5, 6, 7, 8, 13, 14, and 15.
- Figure 3: Map of the plasmid pMTL1.3 containing the 1.3 kb 3' end of the GBSS cDNA used in the preparation of the plasmids of Figure 4, 5, 6, 7, 8, 13, 14, and 15.
- Figure 4.: GBSSI gene silencing vector pKGBA50-IR1.3 with nptII selection marker gene containing the 1.3 kb 3' end of the GBSS cDNA in an inverted repeat configuration interrupted by the 1.1 kb 5' end of the GBSS cDNA.
- Figure 5.: GBSSI gene silencing vector pKGBA50-DR1.3 with nptll selection marker gene containing the 1.3 kb 3' end of the GBSS cDNA in a tandem repeat configuration interrupted by the 1.1 kb 5' end of the GBSS cDNA.
- Figure 6.: GBSSI gene silencing vector pKGBA50-IR1.1 with nptII selection marker gene containing the 1.1 kb 5' end of the GBSS cDNA in an inverted repeat configuration interrupted by the 1.3 kb 3' end of the GBSS cDNA.
- Figure 7.: GBSSI gene silencing vector pKGBA50-DR1.1 with nptII selection marker gene containing the 1.1 kb 5' end of the GBSS cDNA in a tandem repeat configuration interrupted by the 1.3 kb 3' end of the GBSS cDNA.
- Figure 8.: Selection marker-free GBSSI gene silencing vector pKGBA50mf-IR1.1 containing the 1.1 kb 5' end of the GBSS cDNA in an inverted repeat configuration interrupted by the 1.3 kb 3' end of the GBSS cDNA.

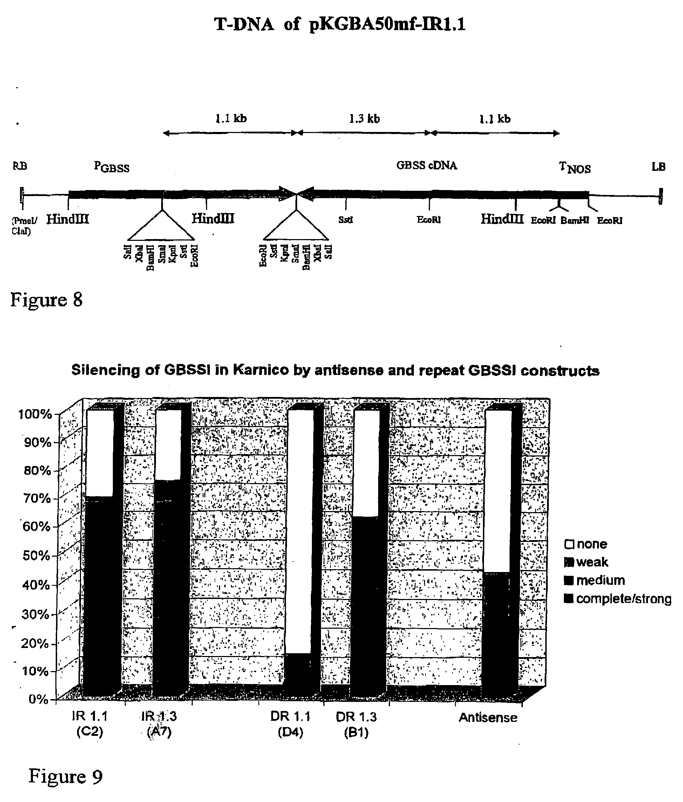

- Figure 9.: Results of transformation of potato plants cv. Karnico with 4 different GBSSI gene silencing constructs containing the GBSSI cDNA in an inverted or tandem repeat configuration comparing to the traditional GBSSI antisense construct. After selection of transformants tuber induction media was added to in vitro grown plants. After 2 to 6 weeks microtubers had developed on most shoots. Starch from cut tuber surfaces was stained with iodine. Transformants having granules staining blue with iodine solution are non-silenced (none), tubers staining red are silenced (completely/strong) and "weak or medium" indicate partially silenced transformants.
- Figure 10.: Map of plasmid pAAP105 mf containing feedback-insensitive potato DHDPS cDNA in control of the GBSSI promoter without plant transformation selection markers.

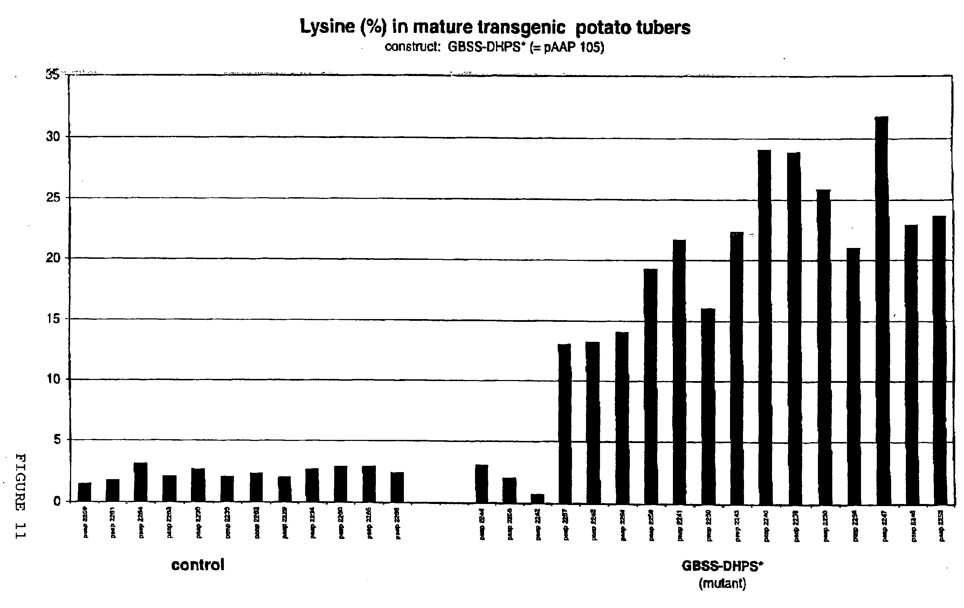

- Figure 11.: Free lysine concentration (% of total free amino acids) of microtubers derived from different independent transformants or control plants. Tubers were induced by adding Tuber induction media to in vitro grown plants. After 2 to 6 weeks microtubers had developed on most shoots. Tissue (0.5-1.0 gram) was homogenised in 2 ml Pi-buffer containing 1 mM dithiothreitol. Nor-leucine was added as an internal standard. Free amino acids were partly purified by extraction with 5 ml of a water:chloroform: methanol mixture (3:5:12). After concentration by lyophilisation to 3 ml, a 20 microl sample was analysed by HPLC using a cation-exchange column (BIOCHROM 20, Amersham Pharmacia biotech).
- Figure 12.: Map of the plasmid pAAP172 containing the 1.1 kb feedback-insensitive potato DHPS* cDNA on control of the potato GBSSI promoter used in the preparation of the plasmids of Figure 13, 14 and 15.
- Figure 13.: Construction of GBSSI gene silencing vector pKGBA50mf R1.1 containing the feedback-insensitive potato DHPS* cDNA of pAAP105.
- Figure 14.: T-DNA of pDHPS-IR1.1 (tandem) containing the 1.1 kb 5' end of the GBSS cDNA in an inverted repeat configuration interrupted by the 1.3 kb 3' end of the GBSS cDNA, and the feedback-insensitive potato DHPS* cDNA in a tandem orientation without plant transformation selection markers.
- Figure 15.: T-DNA of pDHPS-IR1.1 (tandem) containing the 1.1 kb 5' end of the GBSS cDNA in an inverted repeat configuration interrupted by the 1.3 kb 3' end of the GBSS cDNA, and the feedback-insensitive potato DHPS* cDNA in a reverse orientation without plant transformation selection markers.

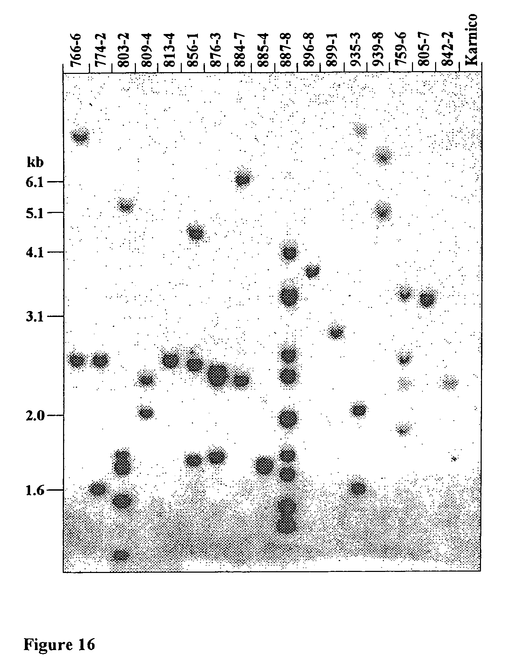

- Figure 16.: Southern blot analysis of DNA isolated from marker-free, vector-free and amylose-free potato transformants. Genomic DNA was isolated from 17 transformants and one untransformed control (Karnico), digested with HindIII and probed with the NOS terminator probe.

### Detailed description

### Optimizing Plant transformation

High-throughput production of marker-free transformants as suggested in this invention calls for high transformation frequencies independent of the genotype. Optimizing transformation protocols for mono- and dicotyledonous plants has been the subject of many studies. For transformation of tuberous plants such as potato, several methods have been published. In many of these protocols the transformation frequencies are very genotype dependent. The essential differences between the different protocols is whether auxins are present during the whole regeneration period or whether a callus initiation phase is followed by a period of absence of auxin but presence of gibberellins. There are also differences between the published protocols in the composition of the media and in the precise levels and types of auxins and cytokinines (Hulme et al. 1992 Plant Molec. Biology 31, 161-167). A number of investigators have shown that ethylene accumulation can inhibit regeneration of potato plants and that this inhibition can be overcome by the presence of ethylene biosynthesis or ethylene action inhibitors (Perl et al. 1988 Plant Cell Rep. 7, 403-406). Accordingly, some protocols add silverthiosulphate (STS) in the regeneration medium. Other modifications can be added to the transformation protocol to increase transformation efficiency. For example, WO 0034491 suggested that reducing moisture conditions to reduce explant weight after Agrobacterium inoculation enhances the transformation efficiency. Possible methods to reduce the weight of the explants during co-culture include increasing the osmotic potential of the medium, addition of desiccants, including calcium oxide or sulphuric acid or air-drying the explants.

For a commercial transformation process it is however convenient to have a single protocol that is efficient for a great range of cultivars. Comparing a number of different protocols on the regeneration of a range of potato cultivars Hulme et al. (1992 Plant Molec. Biology 31, 161-167) conclude that the protocol of Hovenkamp-Hermelink et al. (1988 Euphytica 39, 213-219) is a superior method to those published so far for a number of potato cultivars. We demonstrate herein that two transformation protocols for regeneration have a significant effect on the number of marker-free transformants obtained.

### Optimizing transformation efficiency

A preferred Agrobacterium strain utilized in a method of the invention is modified to contain a gene or genes of interest, or a nucleic acid to be expressed in the transformed cells. The nucleic acid to be transferred is incorporated into the T-region and is flanked by at least one T-DNA border sequence. A variety of Agrobacterium species are known in the art particularly for dicotyledon transformation including *Agrobacterium tumefaciens* and *Agrobacterium rhizogenes.* See, for example, Hooykaas, P.J. 1989 Plant Mol. Siol. 13:327; Smith et al. 1995 Crop Science 35:301; Chilton, M.O. 1993 Proc. Natl. Acad.Sci. USA 90:3119; 19:148; Ishida et al. 1996 Nature Biotechnol. 14:745; Komari, T. et al. 1996 The Plant Journal 10:165. In the Ti plasmid, the T-region is distinct from the vir region whose functions are responsible for transfer and integration. Binary vector systems have been developed where the manipulated T-DNA carrying foreign DNA and the vir functions are present on separate plasmids. In this manner, a modified T-DNA region comprising foreign DNA (the desired nucleic acid to be transferred) is constructed in a small plasmid which replicates in *E. coli.* This plasmid is transferred conjugatively in a tri-parental mating or by electroporation or by freeze-thaw procedure into *A. tumefaciens* which contains a compatible plasmid carrying virulence genes. The vir functions are supplied in trans to transfer the T-DNA into the plant genome. The transformation efficiency is largely influenced by the existence of a super-virulent vir region. Preferred is an *Agrobacterium tumefaciens* strain of the agropine, binary type. Especially preferred is the publicly available *Agrobacterium tumefaciens* strain EHA101, AGL0 or AGL1. These strains contain a C58 bacterial chromosome and a disarmed derivative of the Ti plasmid referred to in the literature as TiBO542 originating from a strongly virulent *Agrobacterium tumefaciens* A281. [See, e.g., Hood E.E. et al, (1986) J. Bacteriology 168:1291-1301, Lazo G.R. et al. (1991) Biotechnology 9: 963-967].

Alternatively, an Agrobacterium strain can be used containing a plasmid equiped for enhanced virulence. Preferred is a superbinary vector to practice the invention with. Such a super-binary vector has been constructed containing a DNA region originating from the virulence region of Ti plasmid pTiBo542 (Jin et al. (1987) J. Bacteriol 169:4417-4425) contained in a super-virulent *Agrobacterium tumefaciens* A281 exhibiting extremely high transformation efficiency (Hood et al. (1984) Biotechnol. 2:702-709; Hood et al. (1986) J. Bacterial. 168:1283-1290; Komari et al. (1986) J. Bacteriol. 166:88-94; Jin et al. (1987) J. Bacterial. 169:4417-4425; Komari T. (1989) Plant Science 60:223-229; ATCC Accession No. 37394).

Super-binary vectors include pTOK162 (Japanese Patent Appl. (Kokai) No. 4222527, EP-A-504,869, EP-A-604,662, and U.S. Patent No. 5,591,616) and pTOK233 (Komari, T. (1990) Plant Cell Reports 9:303-306; and Ishida et al. (1996) Nature Biotechnology 14:745). Super-binary vector pTOK162 is capable of replication in both *E*. *coli* and in *A. tumefaciens.* Additionally, the vector contains the virB, virC, and virG genes from the virulence region of pTiBo542. The plasmids were introduced into the Agrobacterium strains by triple cross (Ditta G. et al., 1980; Proc. Natl. Acad. Sci. USA 77: 7347-7351). Besides the type of Agrobacterium strain, it is known that supplementation of the Agrobacterium suspension with a phenolic compound such as acetosyringone can enhance the transformation efficiency (see Townsend, J. A. et al., US 5,563,055). Typical concentration ranges have been in the range of 100 to 200 microM.

### Optimizing the gene construct

The inhibition of expression of a target gene, commonly referred to as "gene silencing" can be achieved by "antisense downregulation" and "sense downregulation" (also, referred to as "cosuppression"). In antisense downregulation, a DNA fragment which is complementary to all or part of an endogenous target gene is inserted into the genome in reverse orientation. While the mechanism has not been fully elucidated, one theory is that transcription of such an antisense gene produces mRNA which is complementary in sequence to the mRNA product transcribed from the endogenous gene. The antisense mRNA then binds with the naturally produced "sense" mRNA to form a duplex which inhibits translation of the natural mRNA to protein.

Antisense downregulation technology is well-established in the art and used routinely in laboratories around the world. Both overexpression and downregulation are achieved by "sense" technology. If a full length copy of the target gene is inserted into the genome a range of phenotypes can be obtained, some overexpressing the target gene, some under-expressing. A population of plants produced by this method may then be screened and individual phenotypes isolated.

Gene silencing can therefore be achieved by inserting into the genome of a target organism an extra copy of the target gene coding sequence which may comprise either the whole or part or be a truncated sequence and may be in sense or antisense orientation. Additionally, intron sequences which can be obtained from the genomic gene sequence may be used in the construction of suppression vectors. There have also been reports of gene silencing being achieved within organisms of both the transgene and the endogenous gene where the only sequence identity is within the promoter regions.

Although antisense and sense downregulation can result in complete silencing of the target gene, the efficiency is generally not very high. A maximum of 25% of the antisense transformants displayed complete silencing, while only about 10% of the transformants obtained with sense constructs showed some level of silencing (Smith et al. 2000 Nature 407: 319-320; Wolters and Visser, 2000 Plant Mol Biol 43: 377-386).

Recently, it has been observed that the inhibition of a selected target gene within an organism is enhanced, when the gene silencing vector includes a inverted repeat of all or part of a polynucleotide region of the target gene (as suggested in WO99/61632, WO98/53083 and WO99/53050).

The inverted repeat sequence may consist of a T-DNA with one promoter driving expression of a sense copy of the cDNA together with another promoter in front of an antisense copy of the same cDNA (Chuang and Meyerowitz, 2000 Proc Natl Acad Sci USA 97: 4985-4990) or the T-DNA may contain a cDNA sequence flanked on both ends by a promoter (LaCount et al. 2000 Mol Biochem Parasit 111: 67-76), or the T-DNA may contain a promoter driving transcription of an inverted repeat of (part of) the cDNA (Hamilton et al. 1998; Smith et al. 2000 Nature 407: 319-320; Wang and Waterhouse, 2000 Wang MB, Waterhouse PM (2000) Plant Mol Biol 43: 67-82) or the promoter (Mette et al. 2000 EMBO J 19: 5194-5201) of the gene to be silenced.

Some inverted repeat constructs are reported to result in 100% of the transformants displaying silencing of the target gene (Smith et al. 2000 Nature 407: 319-320) in case of using an intron as a spacer between the repeats. The spacer fragment contributes to the stability of the perfect inverted repeat sequences, but is not required for the specificity of the silencing.

Here we demonstrate that more than 60% of potato plants transformed with a GBSSI antisense gene containing an additional, upstream inverted copy of its 5' region, exhibited substantially or even strongly reduced GBSSI activity compared to wild type plants. Only 14% of plants transformed with a similar construct, without the inverted repeat, had reduced GBSSI activity.

Although the mechanism by which the invention operates is not fully understood, we believe that creation of an inverted repeat may yield doublestranded RNA, derived from a DNA sequence showing homology to the gene to be silenced. This, in turn, is thought to trigger the degradation of the endogeneous ssRNA transcripted from the gene to be silenced Based on studies of others (Hamilton AJ et al. (1998) Plant J 15: 737-746; Stam, M (1997) Annals of Botany 79: 3-12) we suspect suppression of GBSS 1 gene expression to be mainly post-transcriptional. Alternatively, Mette MF, et al.(2000) EMBO J 19: 5194-5201) describes a T-DNA containing a promoter driving transcription of an inverted repeat of (part of) the promoter (Mette et al. 2000 EMBO J 19: 5194-5201) of the gene to be silenced resulting in transcriptional silencing and promoter methylation.

The level of over-expression of a target gene in plants can be influenced by many factors. One factor is the choice of transcriptional promoters used. A range of plant compatible promoters are available including tissue specific and inducible promoters. Some of the better documented constitutive promoters include the CaMV 35S promoter and tandem arrangements of this promoter, as suggested in European patent application No. 0 342 926. Yet other factors that can be manipulated to control levels of expression are the presence of transcriptional modification factors such as introns, polyadenylation and transcription termination sites. At the translational level, other factors to consider are the ribosomal binding site and the codon bias of the gene. A translational enhancer sequence derived from the untranslated leader sequence from the mRNA of the coat protein gene of alfalfa mosaic virus coat protein gene placed between a promoter and the target gene has been shown to increase translational efficiency as suggested in US6037527. The construct of the invention may also comprise one or more sequences that encode signal proteins, including pre-, pro- or prepro-sequences. These usually precede the sequence, such that the target gene is expressed as a fusion with the signal proteins. The signal sequence may ensure any post-translational modifications required for the formation of the mature fusion and/or may specifically direct the expressed fusion product to a desired part or organel within the plant or plant cell.

Furthermore, it has been observed that the same construct inserted at different loci on the genome can vary in the level of expression in plants. The effect is believed to be due at least in part to the position of the gene on the chromosome, i.e., individual isolates will have different expression levels. This phenomenon, referred to as "position effect" variation, is the variation in expression of the same gene in independent transformants. The use of naturally occurring DNA sequences called matrix attachment regions or scaffold attachment regions to combat this problem was proposed in U. S. Patent 5,773,689 and in WO 94/24293. WO0032800 showed that Matrix Attachment Regions could enhance expression in plant species representing both monocotyledonous and dicotyledonous plants.

In a preferred embodiment, the promoter that controls gene expression is strong and tissue-specific. Examples of known tissue-preferred promoters include the tuber-directed class I patatin promoter, (Bevan et al., (1986) Nucleic Acids Res. 14: 4625-38), the promoters associated with potato tuber GBSSI gene (Visser et al. 1991 Plant Molec. Biol. 17, 691-699), the soybean promoter of beta-conglycinin, also known as the 7S protein, which drives seed-directed transcription (Bray 1987 Planta 172: 364-370) and seed-directed promoters from the zein genes of maize endosperm (Pedersen et al., 1982 Cell 29: 1015-26).

Alternatively, the target gene can be under the transcriptional control of the CaMV 35S promoter. In this construct, transcriptional activity can be enhanced by a DNA fragment representing part of the CaMV 35S promoter being placed in a direct repeat tandem arrangement.

### Selection of single-copy and vector DNA-free transformants

After transformation, the T-DNA is present in the genome of the host plant as single or multiple, copies. Furthermore, there is information that also DNA beyond the borders is sometimes integrated into the genome of the host plants. This is reported to be the case from 20-30% of the transgenic plants (Martineau, B et al. The Plant Cell 6, 1032-1033, 1994) to 75% in tobacco transformants (Kononov, M.E. et al., The Plant J. 11, 945-957). Registration authorities dealing with requests for (market) registration of transgenic plants and/or transgenic food, are of the opinion that transgenic plants with selection markers, vector DNA and multiple copies / inserts of the T-DNA should be avoided as much as possible. In the present embodiment, transformants free of backbone vector DNA are selected by PCR or Southern blotting. Alternatively the transfer of vector DNA to the plant can be inhibited by inserting a coding sequence outside the T-borders which sequence encodes a (poly)peptide that is toxic to the plant so there is counterselection for plants with superfluous vector-DNA (as suggested in WO99/01563).

### Example 1

### Optimizing the Gene Constructs for efficient inhibition of GBSSI expression

### Gene Constructs

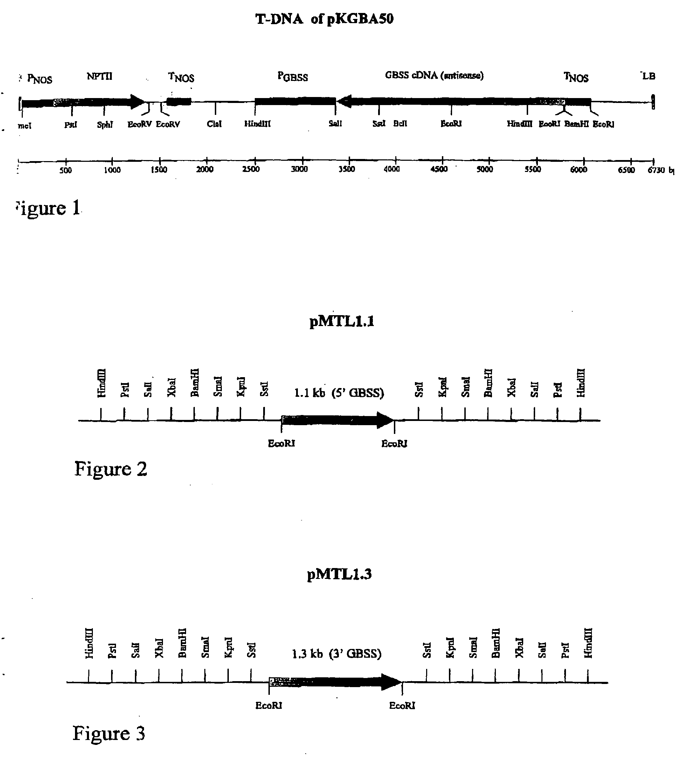

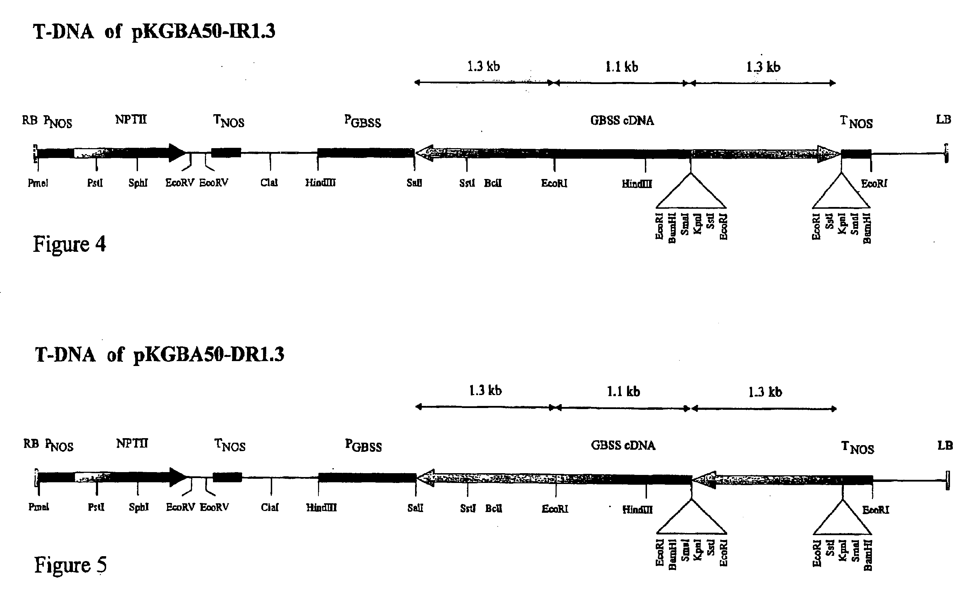

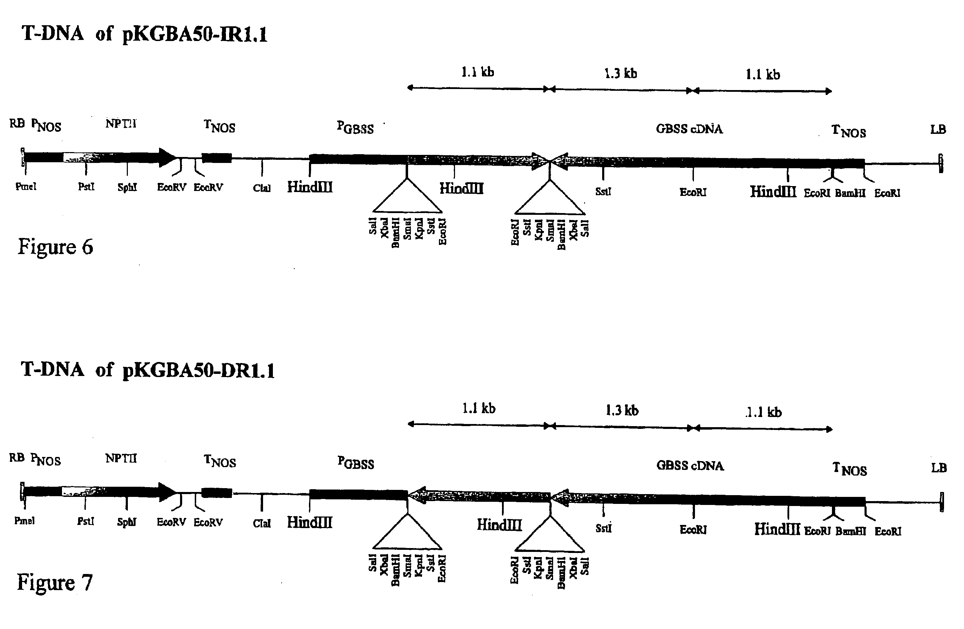

The antisense GBSSI construct pKGBA50 (Figure 1) has been described by Kuipers et al. (1995) Mol Gen Genet 246:745-755. It contains the 2.4-kb GBSSI cDNA in antisense orientation behind the GBSSI promoter in addition to selection marker gene NPTII. Two fragments of the GBSSI cDNA, the 1.1-kb 5' part and the 1.3-kb 3' part, were cloned as EcoRI fragments in plasmids pWx1.1 and pWx1.3, respectively (Visser et al. 1991 Mol Gen Genet 225:289-296). These EcoRI fragments were subcloned into vector pMTL25 (Chambers et al. 1988 Gene 68: 139-149), resulting in plasmids pMTL1.1 and pMTL1.3, see Figures 2 and 3. Plasmid pMTL1.3 was digested with BamHI. The ±1.3-kb BamHI fragment was cloned into BamHI-digested pKGBA50. This yielded two new binary vectors: pKGBA50-IR1.3 (Figure 4), with an inverted repeat of the 1.3-kb fragment, and pKGBA50-DR1.3 (Figure 5), containing a direct repeat of the 1.3-kb fragment. Plasmid pMTL1.1 was digested with SalI. The ±1.2-kb SalI fragment was cloned into SalI-digested pKGBA50. This yielded two additional binary vectors: pKGBA50-IR1.1 (Figure 6), containing an inverted repeat of the 1.1-kb fragment, and pKGBA50-DR1.1 (Figure 7), with a direct repeat of the 1.1-kb fragment.

All constructs were transformed into *E. coli* DH5α (GIBCO BRL). The binary vectors pKGBA50 IR1.3, pKGBA50-DR1.3, pKGBA50-IR1.1, pKGBA50-DR1.1, and pKGBA50-IR1.1 were transformed into *Agrobacterium tumefaciens* LBA4404 (pAL4404) (Hoekema et al. 1983 Nature 303:179-180) by triparental mating, using helper plasmid pRK2013 in *E. coli* HB101 (Figurski and Helinski, 1979 Proc Natl Acad Sci USA 76: 1648-1652).

### Transformation of potato

Potato cultivar 'Karnico' (Anonymous, 1994 Beschrijvende Rassenlijst voor Landbouwgewassen CPRO-DLO, Wageningen, The Netherlands, 342 pp.) had previously been transformed with antisense construct pKGBA50 (Kuipers et al. 1995 Mol Gen Genet 246:745-755). This same potato cultivar was transformed with pKGBA50-IR1.3 (construct A7), pKGBA50-DR1.3 (construct B1), pKGBA50-IR1.1 (construct C2) and pKGBA50-DR1.1 (construct D4). Internodal cuttings from *in vitro* grown plants were used for transformation by *Agrobacterium tumefaciens* co-cultivation, according to the protocol described by Visser RGF (1991) In: K Lindsey (Ed) Plant Tissue Culture Manual, Kluwer Academic Publishers, Dordrecht/Boston/London, pp. B5: 1-9.

Transformants were selected on MS20 medium (Murashige and Skoog, 1962 Physiol Plant 15: 473-497) with 20 g/L sucrose, containing 100 mg/L kanamycin.

### In vitro tuberization

Shoots were transferred to pots with 50 ml solidified MS30 medium. After 3-4 weeks 20 ml of a liquid medium was added, consisting of KI medium ('knolinducerend' (= tuber inducing) medium, Duchefa) with 325 g/L sucrose and 1.75 g/L CCC (chlorocholine chloride, or cycocel.). The pots were placed in a dark growth chamber at a temperature of 18°C. After 2-6 weeks microtubers had developed on most shoots.

### Starch staining

Microtubers were cut and stained with an iodine solution (1.7% (w/v) iodine and 3.3% (w/v) potassium iodide solution), to assess the presence of amylose in the starch granules. Staining of the starch granules was inspected with a microscope.

### DNA analyses

DNA was isolated from *in vitro* shoots by a CTAB DNA isolation protocol as described by Rogers and Bendich (1988) Plant Molecular Biology Manual A6. Kluwer Academic Publ, Dordrecht, pp 1-10. PCR was performed with primers NPT3 and NPT4 (Sequence nos. 3 and 4), to check the presence of the NPTII selection marker gene. Subsequently, PCRs were performed with primers NPT1 and NPT2, trfA1 and trfA2, insB1 and insB2 (Sequence nos. 1,2,5,6,7 and 8; Wolters et al. 1998 Mol Breeding 4: 343-358), to study the presence of backbone vector DNA in the transformants.

Four µg of DNA of the transformants was digested with HindIII, fragments were separated by gel electrophoresis, and Southern blots were made. Blots were hybridized with an NPTII probe, consisting of a NPT3 + NPT4 PCR product from pBI101 (Jefferson et al. 1987 EMBO J 6: 3901-3907), to check the number of T-DNA insertions.

### Efficiency of silencing

The number of transformants per construct, from which microtubers were obtained, are shown in Table 1. The iodine staining results are presented in Table 1 and Figure 9. As a reference the results obtained with antisense construct pKGBA50 are included. Fourteen percent of the transformants obtained with antisense construct pKGBA50 showed complete inhibition of GBSSI activity, as indicated by the red colour of starch granules stained with iodine solution. The direct repeat constructs pKGBA50-DR1.3 (B1) and pKGBA50-DR1.1 (D4) yielded a lower percentage (2-6%) of completely silenced transformants than the antisense construct. In contrast, the inverted repeat constructs pKGBA50-IR1.3 (A7) and pKGBA50-IR1.1 (C2) resulted in a higher percentage of transformants displaying complete inhibition of GBSSI, compared with the antisense construct: 20% for A7 and 62% for C2. Thus, the inverted repeat construct pKGBA50-IR1.1 (C2) was 4.5 times more efficient in completely silencing GBSSI activity than the antisense construct pKGBA50. Inverted repeat construct C2 was much more efficient than construct A7: most (62%) of the C2 transformants showed strong or complete silencing, whereas a large proportion of the A7 transformants (47%) displayed a medium level of silencing. This suggests that either the region of the coding sequence (5' or 3' region) that is present in the inverted repeat is of importance, or that the orientation of the inverted repeat sequences relative to the promoter (antisense DNA - spacer - sense DNA vs. sense DNA - spacer - antisense DNA) is an efficiency determining factor. Similarly, there is a difference between direct repeat constructs B1 and D4; most (51%) of the B1 transformants displayed a low level of silencing, whereas a majority of the D4 transformants (85%) showed no silencing at all. Thus, the 1.1-kb region of the GBSSI cDNA was most efficient for silencing when present in an inverted repeat orientation, whereas it was least efficient when present in a direct repeat orientation. This suggests that it is important to look for the optimal sequence in order to make an inverted repeat construct that results in the highest silencing efficiency.

### Molecular analysis

DNA was isolated from 20 A7 transformants. All showed the expected NPTII PCR fragment, indicating that all contained at least one T-DNA insertion. Nine out of 20 showed the presence of non-T-DNA vector DNA, as determined by PCR of the NPTIII and trfA genes (Table 2). Thus, approximately half of the transformants did not contain backbone vector DNA.

DNA was isolated from 40 C2 transformants displaying strong to complete silencing of GBSSI. All showed the expected PCR fragment with NPTII primers. PCR analyses with backbone vector DNA primers demonstrated that 17 of these transformants contained non-T-DNA sequences, whereas 23 were negative (Table 3). Southern blot analysis of HindIII-digested DNA hybridized with an NPTII probe showed that 5 out of 20 transformants contained a single T-DNA insertion. Four of these had no backbone vector DNA. Thus, it was demonstrated that with the inverted repeat construct pKGBA50-IR1.1 it was possible to obtain completely silenced transformants with a single T-DNA insertion and no backbone vector DNA.

### Example 2

### Isolation of selection marker gene-free GBSSI-silenced transformants

### Gene constructs

Example 1 showed that the 1.1-kb region of the GBSSI cDNA was most efficient for silencing when present in an inverted repeat orientation and that it is possible with this construct (pKGBA50-IR1.1) to obtain completely silenced transformants with a single T-DNA insertion and no backbone vector DNA.

In the previous example transformants were selected on kanamycin using the selectable marker gene NPTII present on construct pKGBA50-IR1.1 To isolate selection marker-free transformants the binary vector pKGBA50-IR1.1 was digested with enzymes PmeI and ClaI (see Figure 1). PmeI yields blunt ends. The ClaI sticky end was made blunt-ended by Klenow polymerase treatment, after which the vector DNA was circularized by blunt end ligation using T4 DNA ligase. This resulted in vector pKGBA50mf-Ik1.1 (marker gene-free) (Figure 8). This construct was transformed into *E*. *coli* DH5α (GIBCO BRL). The binary vector pKGBA50mf-IR1.1 was transformed into *Agrobacterium tumefaciens* LBA4404 (pAL4404) (Hoekema et al. 1983) by triparental mating, using helper plasmid pRK2013 in *E*. *coli* HB101 (Figurski and Helinski, 1979). and into *A. tumefaciens* strain Agl-0 (Lazo et al. 1991). Strain Agl-0 is known to be much more virulent than LBA4404.

### Transformation of potato and selection of transformants

Internodal cuttings from *in vitro* grown plants of potato cultivar 'Karnico' were used for transformation by *Agrobacterium tumefaciens* co-cultivation, according to the protocol described by Visser (1991). Potato cultivar 'Karnico' was transformed with pKGBA50mf-IR1.1 in *A. tumefaciens* LBA4404 or in *A*. *tumefaciens* Agl-0, according to the same protocol. No selection was performed. After four weeks the first shoots were harvested and harvesting of shoots continued for about three months. No more than two regenerants per stem explant were harvested and shoots were allowed to grow on MS20 medium. After 1 to 2 weeks leaf or stem material of 8 or more independent shoots was harvested and pooled (pool size depended on the frequency of transformants expected). DNA of these pools of shoots was isolated in 96-wells microtiter plates using the Magnesil genomic DNA isolation kit purchased from Promega. PCR analyis was performed on DNA isolated from the pools of regenerants with the primers BINMCS and GBSS-0 (Sequence nos. 9 and 10), to check for the presence of transformants. Of the PCR positive pools leaf and / or stem material of individual shoots was harvested in 96-wells microtiter plates and genomic DNA was isolated using the Magnesil genomic DNA isolation kit purchased from Promega. PCR analyis was performed on DNA isolated from the individual regenerants with the primers BINMCS and GBSS-0 (Sequence nos. 9 and 10), to check for the presence of transformants.

### In vitro tuberization

To PCR-positive shoots 20 ml of a liquid medium was added, consisting of KI medium ('knolinducerend' (= tuber inducing) medium, Duchefa) with 325 g/L sucrose and 1.75 g/L CCC (chlorocholine chloride, or cycocel.). The pots were placed in a dark growth chamber at a temperature of 18°C. After 2 to 6 weeks microtubers had developed on most shoots.

### Starch staining

Microtubers were cut and stained with an iodine solution to assess the presence of amylose in the starch granules. Staining of the starch granules was inspected with a microscope.

### DNA analyses

DNA was isolated from in vitro shoots by a CTAB DNA isolation protocol as described by Rogers and Bendich (1988). PCRs were performed with primers NPT1 and NPT2, trfA1 and trfA2, insB1 and insB2 (Sequence nos. 1,2,5,6,7 and 8; Wolters et al. 1998 Mol Breeding 4: 343-358), to study the presence of backbone vector DNA in the transformants. Four µg of DNA of the transformants was digested with HindIII, fragments were separated by gel electrophoresis, and Southern blots were made. Blots were hybridized with a NOS terminator probe, to check the number of T-DNA insertions.

### Production adventitious shoots

Of the marker-free amylose-free vector-DNA free genotypes adventitious shoots were produced according to Hovenkamp-Hermelink et al. (1988). The leaves of 10 to 20 sterile tissue cultured shoots per genotype were floated on a solution of 147 mg/l CaCl₂.2H2O and 80 mg/l NH₄NO₃, supplemented with 10 mg/l BAP and 10 mg/l NAA. After one night floating (16 hrs), the leaves were cut into explants of 3-4 mm width, 100 explants per genotype and placed on a callus induction MS medium with 40 g/l mannitol, 10 g/l sucrose, 2.25 mg/l BAP, 0.0175 mg/l IAA and 8 g/l agar. After six days explants were transferred to MS medium supplemented 15 g/l sucrose, 2.25 mg/l BAP, 5 mg/l GA₃ and 8 g/l agar for regeneration. The explants were transferred to fresh regeneration medium every three weeks. Adventitious shoots (2-4 per explant) were harvested, harvesting at least 100 shoots per genotype and were grown in containers on MS medium with 20 g/l sucrose. When shoots had formed more than four nodes they were subjected to in vitro tuberization and screened for amylose-free starch as described above.

### Efficiency of marker-free transformation

In the course of five independent experiments approximately 8,000 stem explants of potato cultivar'Karnico' were inoculated with either pKGBA50mf-IR1.1 in *Agrobacterium tumefaciens* LBA4404 or AGL-0. Over a period of one to four months after inoculation one or two regenerated shoots per explant were harvested and were allowed to grow on Murashige and Skoog medium. After 1 or 2 weeks leaf or stem material of at least 8 independent shoots was harvested and pooled. DNA of these pools of shoots was isolated in 96-wells microtiter plates. Using a polymerase chain reaction (PCR)-based approach, we tested for the presence of transformants in the pools of shoots. Of the PCR positive pools, analyses were performed on DNA isolated from the individual regenerated plants, to check for the presence of transformants. After transformation with LBA4404 less than 0.2% of the harvested shoots were scored PCR-positive, whereas with AGL-0 this percentage was 4.6% (Table 4). The frequency of PCR-positive transformants of five independent transformation experiments with AGL-0 was between 1.3 and 5.6%, whereas for LBA 4404 this varied from 0 to 0.8%.

To determine the frequency of PCR-positive shoots showing the desired amylose-free phenotype, shoots were grown in vitro under high sucrose conditions to induce tubers. Developed microtubers were stained with iodine and the starch granules microscopically assessed for the presence of amylose. Of 220 PCR-positive transformants analysed approximately 45% showed complete inhibition of GBSSI activity, as indicated by the red colour of the starch granule. This percentage is somewhat lower than the 60% amylose-free plants we obtained with pKGBA50mf-IR1.1 using the NptII as selection marker, which suggests that occasionally we might select a false positive with the PCR detection system.

The results, however, strongly indicate that it is feasible to select transformants without the use of marker genes and that the efficiency of obtaining marker-free transformants is improved by using a virulent Agrobacterium strain and an optimised gene-silencing construct.

### Selection of single-copy and vector DNA-free transformants

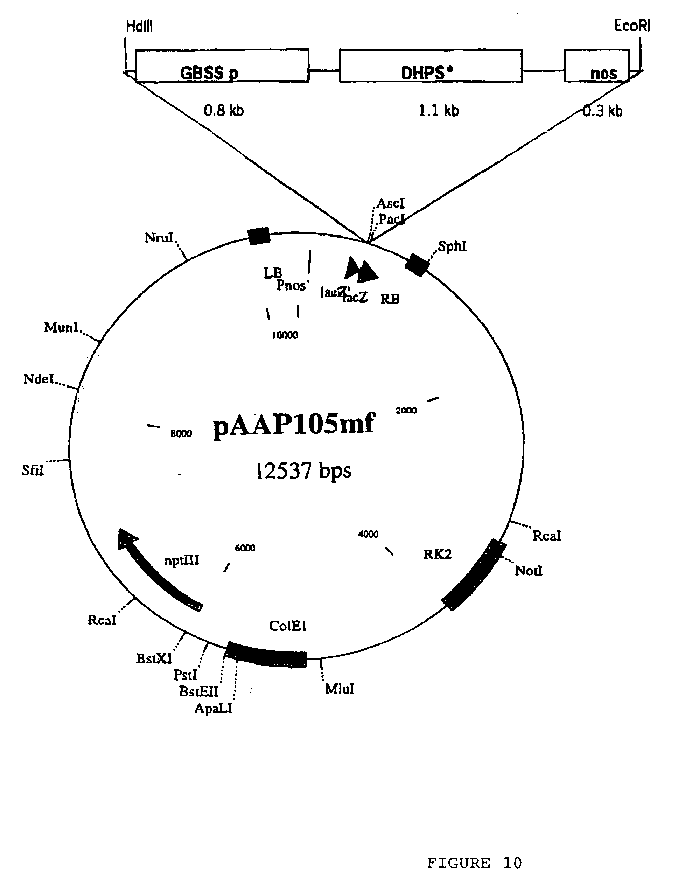

Because of all the controversy regarding the incorporation of DNA other than the gene of interest transformants should be isolated which do not possess vector DNA and multiple inserts of the T-DNA. Integration of DNA beyond the borders into the genome of the host plants is reported to occur in 20 to 75% of the transformed plants. Selected marker-free amylose-free potato transformants were analysed for presence of backbone vector DNA by PCR using primers to five open reading frames of the pBIN19 vector. Of the 99 tested amylose-free transformants 39 were negative for all five DNA fragments. These 39 vector DNA-free transformants were further analysed by Southern blot hybridisation using the NOS terminator as probe (Fig. 10). These analyses showed that most (30) transformants contain 3 or less copies of the T-DNA insertion. Moreover, of the 39 transformants analysed 14 contained a single T-DNA insertion.

Thus, it is demonstrated that it is feasible to obtain transformants without the use of selection marker genes free of backbone vector DNA, containing only one insert of the T-DNA, in which an endogenous gene is completely silenced.

### Chimerism

To investigate whether the PCR-selected transformants are genetic chimeras due to regeneration from more than one cell, we stained the surface of cut tubers with an iodine solution for uniform inhibition of amylose synthesis. During the staining of one tuber per independent transformant no sectorial staining was observed. All tubers were either completely red or completely blue. Staining of more than twenty tubers each of sixty independent transformants with iodine showed only one line with red and blue sectors of cells. These observations indicate that chimerism is occurring, however, at a very low frequency.

Furthermore, 28 independent marker-free amylose-free and vector-DNA free transformants were screened for chimeraes using the adventitious shoot method. More than 100 adventitious shoots were generated per genotype and each of the shoots were subjected to in vitro tuberization and were screened for the amylose-free phenotype. After screening more than 3000 shoots we have not found one adventitious shoot which formed a microtuber containing amylose. This demonstrates that our marker-free transformation method do not or at very low rate result in chimeric plants.

### Example 3

### Effect of transformation method on the efficiency of obtaining marker-free transformants

To determine the effect of the transformation method on the efficiency of marker-free transformation two transformation protocols were tested.

Potato plants cv. Karnico were transformed by co-cultivation of explants with *Agrobacterium tumefaciens* AGL0 containing plant transformation vector pKGBA50mf-IR1.1.

### Transformation protocol 1.1 (Visser, 1991)

Shoot tips of potato cultivar Karnico were subcultured on media containing MS major and minor salts, 3% sucrose, 0.8% agar at pH 5.6. Cultures are grown for 4 weeks at 21 DEG C. in a 16 hour photoperiod. Stem internodes are cut into 2 to 5mm lengths and placed on two layers of filterpaper on solid R3B media for 1 day before co-cultivation. The R3B medium used contained the salts and vitamins of MS medium (4.71 g/l) plus 3% saccharose, 2 mg/l NAA, 1 mg/l BAP and 0.8% agar, pH 5.8. The layers of filterpaper was covered with 2 ml of PACM liquid media consisting of MS (4,71 g/l), 2.0 g/l caseine hydrolysate, 3% saccharose, 1 mg/L 2,4 D and 0.5 mg/L kinetine, pH 6.5. Co-cultivation was carried out for 5 to 10 minutes in a two-day culture of Agrobacterium before explants were blotted on filter paper to remove excess bacteria. After blotting explants were transferred back on the same petriplates and incubated for two days at 21 DEG C. in a 16 hours photoperiod.

After two days the explants were transferred to Zcvh media consisting of 4.71 g/l MS, 2.0% saccharose, 0.8% agar, 200 mg/l cefotaxime, 200 mg/l vancomycine and 1 mg/l zeatine. Cultures were transferred every two weeks to fresh Zcvh media. After four weeks the first shoots were harvested and transferred to media containing MS major and minor salts, 3% sucrose, 0.8% agar at pH 5.6. No more than two shoots per explant were harvested and harvesting continued for approximately 3 months.

### Transformation protocol 2. (Edwards et al. Plant Molec. Biol. 17: 89-100, 1991)

Shoot tips of potato cultivar Karnico were subcultured on media containing MS major and minor salts, 3% sucrose, 0.8% agar at pH 5.6. Cultures are grown for 4 weeks at 21 DEG C. in a 16 hours photoperiod.

Stem internodes are cut into 2 to 5mm lengths and placed on two layers of filterpaper on solid R3B media for 1 day before co-cultivation. The R3B medium used contained the salts and vitamins of MS medium (4.71 g/l) plus 3% saccharose, 2 mg/l NAA, 1 mg/l BAP and 0.8% agar, pH 5.8. The layers of filterpaper was covered with 2 ml of PACM liquid media consisting of MS (4,71 g/l 2.0 g/l caseine hydrolysate, 3% saccharose, 1 mg/L 2,4 D and 0.5 mg/L kinetine, pH 5.8. Co-cultivation was carried out for 30 minutes in a two-day culture of Agrobacterium before explants were blotted on filter paper to remove excess bacteria. After blotting explants were transferred to solid PCM media consisting of 4.71 g/l MS, 2.0% saccharose, 0.8% agar, 2 mg/l 2,4 D and 0.5 mg/l zeatine, pH 5.8 and incubated for two days in the dark at 21 DEG C. After two days the explants were transferred to PCM media containing 200 mg/l cefotaxime and incubated for four days at 21 DEG C. in a 16 hours photoperiod.

After these four days explants were transferred to solid PSM media consisting of 4.71 g/l MS, 2.0% saccharose, 0.8% agar, 2 mg/l GA₃, 1.0 mg/l zeatine, pH 5.8 and 200 mg/l cefotaxime. Cultures were transferred every three weeks to fresh PSM media containing 200 mg/l cefotaxime. After four weeks the first shoots were harvested and transferred to media containing MS major and minor salts, 3% sucrose, 0.8% agar at pH 5.6. No more than two shoots per explant were harvested and harvesting continued for approximately 3 months.

After 1 to 2 weeks leaf or stem material of 8 or more independent shoots was harvested and pooled (pool size depended on the frequency of transformants expected). DNA of these pools of shoots was isolated in 96-wells microtiter plates using the Magnesil genomic DNA isolation kit purchased from Promega. PCR analyis was performed on DNA isolated from the pools of regenerants with the primers BINMCS and GBSS-0 (Sequence nos. 9 and 10), to check for the presence of transformants. Of the PCR positive pools leaf and / or stem material of individual shoots was harvested in 96-wells microtiter plates and genomic DNA was isolated using the Magnesil genomic DNA isolation kit purchased from Promega. PCR analyis was performed on DNA isolated from the individual regenerants with the primers BINMCS and GBSS-0 (Sequence nos. 9 and 10), to check for the presence of transformants.

### Results

Two independent transformations of 1,000 stem explants of potato cultivar 'Karnico' were carried out using transformation protocol 1 and 2. The frequency of explants from which a shoot is harvested is comparable between the two transformation protocols. Protocol 1 resulted in 74% explantants with shoots whereas with protocol 2 67% of explantants formed one or more shoots. The frequency of PCR-positive shoots obtained was however very different between the two methods. With protocol 2 between 0.6 % and 1.0 % of the harvested shoots were scored PCR positive, whereas with protocol 1 this percentage was between 5.5% and 6.0%.

Thus, the use of an optimised transformation protocol may result in 5 to 10 times the number of PCR positive shoots and increases efficiency of marker-free transformation.

### Example 4

### Marker-free selection of high-lysine potato transformants

### Gene constructs

A feedback insensitive potato DHPS was created by changing the evolutionary conserved amino acid residue 134 (asparagine) into a cysteine residue, as suggested in EP99204520. The chimeric gene containing the mutant DHPS gene was constructed by subcloning DHPS cDNA from the pTriplex vector (pAAP42) first in pCR-Script SK(+) (pAAP55) and from this vector as a XbaI-Eco RI fragment in the pBluescript SK vector digested with XbaI-EcoR (pAAP57). At the 5'end the mutated DHPS cDNA was fused to a HindIII-SalI fragment of the 800 bp long GBSSI promoter fragment. Downstream of the mutant DHPS sequence the termination signal of the nopaline synthase gene from *Agrobacterium. tumefaciens* was inserted (Greve, H.D. et al. (1983) J.Mol.Appl.Genet. 1: 499-511) as an SstI-EcoRI fragment. The complete chimeric gene was subcloned into the HindIII-EcoRI sites of pBINPLUS which was first digested with ClaI and partially with RcaI and religated to remove NPTII selection marker (Van Engelen, F.A. et al. (1995) Transgenic Research 4: 288-290). This resulted in vector pAAP105mf (Figure 10).

All constructs were transferred into *E. coli* DH5α (GIBCO BRL).

### Transformation of potato plants

The binary vector pAAP105mf was used for freeze-thaw transformation of *Agrobacterium tumefaciens* strain AGL0 (Höfgen, R. and Willmitzer, L. (1988) Nucl.Acids Res. 16: 9877). Transformed AGL0 was subsequently used for inoculation of potato (*Solanum tuberosum,* variety Karnico) stem explants. No selection was performed. After four weeks the first shoots were harvested and harvesting of shoots continued for about three months. No more than two regenerants per stem explant were harvested and shoots were allowed to grow on MS20 medium. After 1 to 2 weeks leaf or stem material of 8 or more independent shoots was harvested and pooled (pool size depended on the frequency of transformants expected). DNA of these pools of shoots was isolated in 96-wells microtiter plates using the Magnesil genomic DNA isolation kit from Promega. PCR analysis was performed on DNA isolated from the pools of regenerants with the primers BINMCS and GBSS-0 (Sequence nos. 9 and 10), to check for the presence of transformants. Of the PCR positive pools leaf and / or stem material of individual shoots was harvested in 96-wells microtiter plates and genomic DNA was isolated using the Magnesil genomic DNA isolation kit from Promega. PCR analyis was performed on DNA isolated from the individual regenerants with the primers BINMCS and GBSS-0 (Sequence nos. 9 and 10), to check for the presence of transformants.

### In vitro tuberization

To PCR-positive shoots 20 ml of a liquid medium was added, consisting of KI medium. The pots were placed in a dark growth chamber at a temperature of 18°C. After 2 to 4 weeks microtubers had developed on most shoots.

### Analysis of free amino acid content in transgenic plants

Tissue (0.5-1.0 gram) was homogenised with mortar and pestle in 2 ml 50 mM Pi-buffer (pH 7.0) containing 1 mM dithiothreitol. Nor-leucine was added as an internal standard. Free amino acids were partly purified by extraction with 5 ml of a water:chloroform:methanol mixture (3:5:12, by volume). Water phase was collected and the organics phase was re-extracted twice. After concentration by lyophilisation to 3 ml, a 20 microliter sample was analysed by HPLC using a cation-exchange column with post-column ninhydrine derivatisation of the amino acids detected at 570 and 440 nm (BIOCHROM 20, Amersham Pharmacia biotech). Figure 11 shows the lysine levels as the percentage of the total amino acids in tubers in 12 'control' untransformed potato plants and in 17 potato plants containing the mutated DHPS gene construct pAAP105mf. The lysine levels increased from 2-2.5% in the control wild type tubers to a maximum percentage of 30 in the transformed tubers, resulting in lysine being a 'bulk' amino acid in stead of a 'low level' essential amino acid.

### Example 5

### Marker-free selection of amylose-free high-lysine potato transformants

### Gene Construct

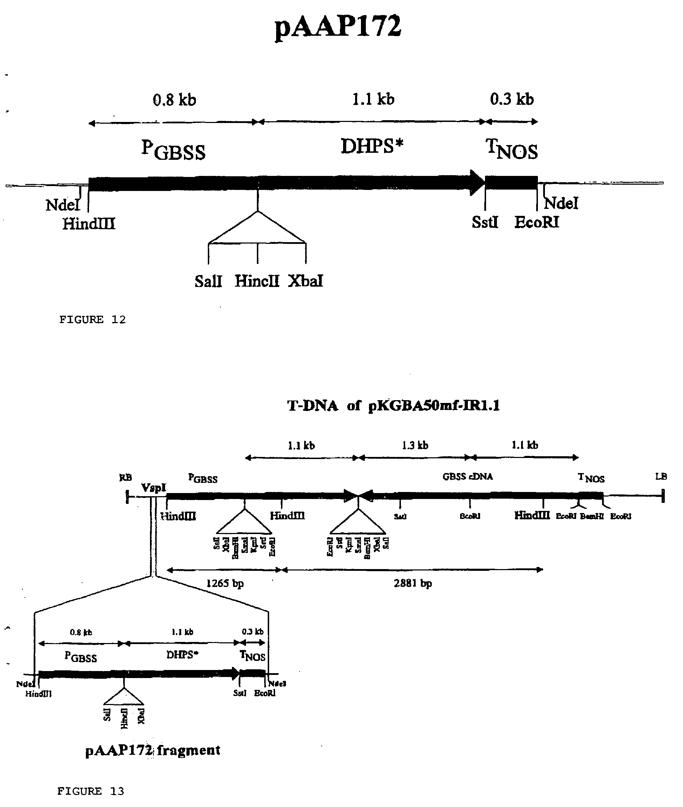

A linker was made (5'-AGCTGCATATGAAGCTTTCTAGATCTGAATTCCATATGT-3' and
3'-CGTATACTTCGAAAGATCTAGACTTAAGGTATACATTAA-5') that contained a HindIII-compatible overhang at the 5' end, and an EcoRI-compatible overhang at the 3' end. This linker was ligated into HindIII/EcoRI-digested pUC28 plasmid (Benes et al. 1993 Gene 130: 151-152), which yielded plasmid pAAP171. Construct pAAP105, the binary vector containing the mutant DHPS gene, was digested with HindIII and EcoRI. The 2.2-kb fragment containing the GBSSI promoter-DHPS gene-NOS terminator was cloned into HindIII/EcoRI-digested pAAP171. This yielded construct pAAP172 (see Figure 12).

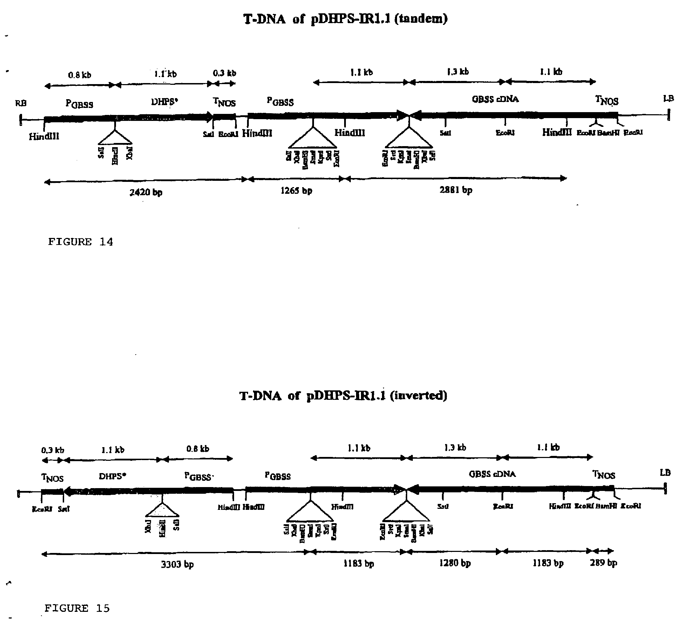

Construct pAAP172 was digested with NdeI and ScaI (a ScaI site is present in the pUC28 backbone). The digested DNA was extracted with phenol/chloroform, and ethanol-precipitated. Binary vector pKGBA50mf-IR1.1 was digested with VspI and treated with alkaline phosphatase, extracted with phenol/chloroform, and ethanol-precipitated. The 2.2-kb NdeI fragment of pAAP172 was ligated into the VspI-digested pKGBA50mf-IR1.1 vector (Figure 13), which yielded two different constructs: pDHPS-IR1.1 (tandem), in which the two GBSSI promoters point in the same direction (Figure 14), and pDHPS-IR1.1 (inverted), in which the two GBSSI promoters point in opposite directions (Figure 15). These constructs were transferred from *E*. *coli* DH5α into *Agrobacterium tumefaciens* Agl-0.

### Transformation of potato and selection of transformants

Internodal cuttings from in vitro grown plants of potato cultivar 'Karnico' were used for transformation by *Agrobacterium tumefaciens* co-cultivation, according to the protocol described by Visser (1991).

Potato cultivar 'Karnico' was transformed with pDHPS-IR1.1 (tandem) and pDHPS-IR1.1 (inverted) in *A. tumefaciens* Agl-0 according to the same protocol. No selection was performed. After four weeks the first shoots were harvested and harvesting of shoots continued for about three months. No more than two regenerants per stem explant were harvested and shoots were allowed to grow on MS20 medium. After 1 to 2 weeks leaf or stem material of 8 or more independent shoots was harvested and pooled (pool size depended on the frequency of transformants expected). DNA of these pools of shoots was isolated in 96-wells microtiter plates using the Magnesil genomic DNA isolation kit from Promega. PCR analyis was performed on DNA isolated from the pools of regenerants with the primers BINMCS and GBSS-0 (Sequence nos. 9 and 10), to check for the presence of transformants. Of the PCR-positive pools leaf and / or stem material of individual shoots was harvested in 96-wells microtiter plates and genomic DNA was isolated using the Magnesil genomic DNA isolation kit from Promega. PCR analyis was performed on DNA isolated from the individual regenerants with the primers BINMCS and GBSS-0 (Sequence nos. 9 and 10), to check for the presence of transformants.

### In vitro tuberization

To PCR-positive shoots 20 ml of a liquid medium was added, consisting of KI medium. The pots were placed in a dark growth chamber at a temperature of 18°C. After 2 to 4 weeks microtubers had developed on most shoots.

### Starch staining

Microtubers were cut and stained with an iodine solution to assess the presence of amylose in the starch granules. Staining of the starch granules was inspected with a microscope.

### Analysis of free amino acid content in transgenic plants

Tissue (0.5-1.0 gram) was homogenised with mortar and pestle in 2 ml 50 mM Pi-buffer (pH 7.0) containing 1 mM dithiothreitol. Nor-leucine was added as an internal standard. Free amino acids were partly purified by extraction with 5 ml of a water:chloroform:methanol mixture (3:5:12). Water phase was collected and the remaining re-extracted twice. After concentration by lyophilisation to 3 ml, a 20 microl sample was analysed by HPLC using a cation-exchange column with post-column ninhydrine derivatisation of the amino acids detected at 570 and 440 nm (BIOCHROM 20, Amersham Pharmacia biotech).

### Example 6

### Marker-free selection of amylose-free cassava transformants

The following part describes an *Agrobacterium tumefaciens* -mediated transformation procedure to produce genetically modified cassava plants of which the starch consists predominantly of amylopectin without the help of selectable marker genes such as nptII, pat, basta, htp or others.

### Gene constructs

PCR primers (sequences nos. 12-20) were designed to amplify parts of the cassava GBSSI cDNA sequence: two nested forward and one reverse primer for each of the following three regions: the 5' part (bases 1-800), the middle part (b. 800-1500), and the 3' part (b. 1200-2000). The forward primers contained an EcoRI restriction site, whereas the reverse primers contained an XbaI restriction site.The PCR products of each of the three parts of the GBSSI cDNA were digested with EcoRI and ligated. The obtained ligation products contained an inverted repeat of part of the cassava GBSSI cDNA, with the region between the two nested forward primers acting as a spacer. This sequence was digested with XbaI and cloned into vector pMTL24 (Chambers et al. 1988). The inverted repeat was removed from this vector by digestion with BamHI, and subsequently ligated between the potato GBSSI promoter and the NOS terminator in BamHI-digested binary vector pKGBA50mf-IR1.1 (see Figure 8). In this way three marker gene-free cassava GBSSI inverted repeat constructs were obtained. These were transformed to *Agrobacterium tumefaciens* strain Agl-0.

### Plant material and tissue culture media used

Plants of the genotype TMS60444 and Adira 4 were maintained by monthly subculture of one node cuttings on medium supplemented with Murashige and Skoog (1962, Physiol. Plant. 15: 473-497.) salts and vitamins and 40 g/l sucrose (MS4). Friable embryogenic callus (FEC) lines were initiated as follows:
- isolation of meristems or immature leaves from donor plants
- culture of meristems/leaves on MS40 supplemented with 6 mg/l NAA and 6 mg/l Picloram
- isolation of compact embryogenic tissue and culture on a medium supplemented with Gresshoff and Doy (1974, Planta 107:161-170) salts and vitamins, 60 g/l sucrose and 10 mg/l Picloram (GD6).
- isolation of FEC (small clumps of aggregated, spherical units) which were cultured on GD6 medium. FEC was maintained by a 3 weeks subculture on GD6 medium. Liquid cultures were initiated by transferring 0.5 g of FEC into flask of 200 ml with 50 ml of liquid medium supplemented with Schenk and Hildebrandt (1972, Canadian Journal of Botany 50:199-204) salts and vitamins, 60 g/l sucrose and 10 mg/l Picloram (SH6). The medium was refreshed twice a week and after 2 weeks the content of a each flask was divided over 5 new flasks. The flasks were cultured on a rotary shaker (LAB-line Instruments Inc. Model 3519) at 120 rpm.

### Infection of FEC with Agrobacterium

Ten samples of 100 mg FEC of TMS604444 were used as starting material. The FEC was spread on medium supplemented with Gresshoff and Doy salts and vitamins, 60g/l sucrose, 10 g/l agar and 10 mg/l picloram (GD6) and a few drops of the Agrobacterium strain Agl-0, containing the GBSSI inverted repeat construct, was added. Two days later the FEC was washed twice with distilled, sterile water to remove excess Agrobacterium. Hereafter the FEC was transferred to liquid medium consisting of Schenk and Hildebrandt salts and vitamins, 60g/l sucrose, 10 g/l agar and 10 mg/l picloram (SH6), supplemented with antibiotics such as cefotaxime to kill Agrobacterium. The medium was refreshed after one week and after two weeks the FEC was spread finely on GD6 medium supplemented with cefotaxime. Two weeks later 50 mg of FEC of the 10 infected samples was evaluated for the integration of transgenes using a PCR based selection system. A small aliquot of FEC material was harvested and DNA of the cells was isolated using the Magnesil genomic DNA isolation kit purchased from Promega. PCR analyis was performed on the DNA isolates with the primers BINMCS and GBSS-0 (Sequence nos. 9 and 10), to check for the presence of transformants. Two of the 10 samples were positive for the GBSS-0/BINMCS primers. These samples were cultured on maturation medium supplemented with cefotaxime. Maturation medium consisted of Murashige salts and vitamins, 40g/l sucrose, 10 g/l agar (MS4) and 1 mg/l picloram. The tissue was transferred every two weeks to fresh maturation medium for a total period of four months. During this period torpedo shaped somatic embryos, developing from the FEC, were isolated and cultured on MS4 medium supplemented with 0.1 mg/l BAP and cefotaxime to allow development into mature somatic embryos. Mature somatic embryos were first cultured for one week on liquid MS4 supplemented with 1.0 mg/l BAP and cefotaxime and then transferred to solid MS4 supplemented with 1.0 mg/l BAP. Shoots developing from the somatic embryos were rooted on MS4 supplemented with cefotaxime. All the rooted plants were subjected to the PCR based selection system. Leaf material of 8 shoots was harvested and pooled. DNA of these pools of shoots was isolated in 96-wells microtiter plates using the Magnesil genomic DNA isolation kit purchased from Promega. PCR analyis was performed on DNA isolated from the pools of regenerants with the primers BINMCS and GBSS-0 (Sequence nos. 9 and 10), to check for the presence of transformants. Of the PCR positive pools leaf and / or stem material of individual shoots was harvested in 96-wells microtiter plates and genomic DNA was isolated using the Magnesil genomic DNA isolation kit purchased from Promega. PCR analysis was performed on DNA isolated from the individual regenerants with the primers BINMCS and GBSS-0 (Sequence nos. 9 and 10), to check for the presence of transformants. In total 1264 and 984 plants were obtained from the two lines of which respectively 5 and 38 plants were PCR positive. The PCR positive plants were grown in the greenhouse in small pots. Three months later the starch was isolated from tubers and analysed for amylose content. In 27 plants the starch was essentially amylose-free.

**Table 1. Levels of silencing in transformants obtained with different constructs.**

| Construct | No. of transformants | Level of silencing of GBSSI | | | |
|---|---|---|---|---|---|
| | | Complete/strong | Medium | Weak | None |
| C2 | 133 | 83 (62%) | 6 (5%) | 2 (2%) | 42(31%) |
| A7 | 118 | 24 (20%) | 55 (47%) | 10 (8 %) | 29 (25%) |
| D4 | 66 | 4 (6%) | 2 (3%) | 4 (6%) | 56 (85%) |
| B1 | 77 | 2 (2 %) | 7 (9%) | 39(51%) | 29 (38%) |
| Antisense | 144 | 20 (14%) | 20 (14%) | 22 (15%) | 82 (57%) |

**Table 2. Molecular analysis of individual A7 transformants.**

| Transformant | Level of silencing | PCR fragments | | |
|---|---|---|---|---|
| | | NPTI I | NPTI II | trfA |
| A7-1 | Weak | + | - | - |
| A7-3 | None | + | - | - |
| A7-7 | Weak | + | - | - |
| A7-11 | None | + | - | - |
| A7-14 | Strong | + | + | + |
| A7-22 | Strong | + | + | + |
| A7-26 | Strong | + | + | + |
| A7-32 | Strong | + | - | - |
| A7-41 | Weak | + | - | - |
| A7-42 | Strong | + | + | + |
| A7-43 | Strong | + | + | + |
| A7-44 | Strong | + | - | - |
| A7-48 | Weak | + | - | - |
| A7-53 | Strong | + | + | + |
| A7-62 | Medium | + | + | + |
| A7-74 | Strong | + | - | - |
| A7-76 | Weak | + | - | - |
| A7-84 | Strong | + | + | - |
| A7-95 | Complete | + | + | - |
| A7-98 | Weak | + | - | - |

**Table 3. Molecular analysis of individual C2 transformants.**

| Transformant | Level of silencing | PCR fragments | | | | No. of T- DNA bands |
|---|---|---|---|---|---|---|
| | | NPTI I | NPTI II | trfA | InsB | |
| C2-1 | Strong | + | + | ND | ND | 4 |
| C2-5 | Medium | + | + | - | - | ND |
| C2-6 | Weak | + | - | - | - | ND |
| C2-7 | Complete | + | + | ND | ND | >5 |
| C2-8 | Complete | + | + | ND | ND | >5 |
| C2-10 | Strong | + | + | ND | ND | 3 |
| C2-11 | Complete | + | + | + | ND | >5 |
| C2-14 | Complete | + | - | - | ND | 3 |
| C2-15 | Complete | + | - | - | ND | 4 |
| C2-16 | Medium | + | - | - | ND | 2 |
| C2-17 | Complete | + | ? | + | ND | 3 |
| C2-20 | Complete | + | - | - | ND | 1 |
| C2-25 | Complete | + | + | ND | ND | 2 |
| C2-29 | Complete | + | - | - | ND | 1 |
| C2-30 | Complete | + | + | ND | ND | >4 |
| C2-33 | Strong | + | - | - | - | ND |
| C2-38 | Strong | + | - | - | ND | 1 |
| C2-41 | Strong | + | - | - | - | ND |
| C2-43 | Strong | + | + | + | - | ND |
| C2-47 | Complete | + | - | + | - | ND |
| C2-65 | Complete | + | + | ND | ND | >4 |
| C2-66 | Complete | + | - | - | ND | 2 |
| C2-70 | Strong | + | - | - | - | ND |
| C2-71 | Complete | + | - | - | ND | 2 |
| C2-75 | Strong | + | - | - | - | ND |
| C2-80 | Strong | + | - | - | - | ND |
| C2-83 | Strong | + | + | ND | ND | 1 |
| C2-85 | Strong | + | + | + | + | ND |
| C2-86 | Medium | + | - | - | - | ND |
| C2-95 | Strong | + | - | - | - | ND |
| C2-112 | Strong | + | + | + | - | ND |
| C2-114 | Complete | + | - | - | ND | 1 |
| C2-116 | Strong | + | + | ND | ND | 4 |
| C2-151 | Strong | + | - | - | - | ND |
| C2-152 | Strong | + | - | - | - | ND |
| C2-156 | Complete | + | - | - | - | ND |
| C2-167 | Strong | + | + | - | + | ND |
| C2-168 | Strong | + | - | - | - | ND |
| C2.170 | Strong | + | - | - | - | ND |
| C2-180 | Strong | + | - | - | - | ND |

| | | | | | | |
|---|---|---|---|---|---|---|
| ND = not determined | | | | | | |

**Table 4. Effect of Agrobacterium strain on transformation efficiency on potato.**

| | | PCR analysis | | Phenotypic analysis | |
|---|---|---|---|---|---|
| | | PCR-positive shoots^{a} | | amylose-free transformants^{b} | |
| Exp. 1, LBA4404 | | 0/1112 | *(0.0)* | 0/0 | *(0.0)* |
| | AGL-0 | 50/888 | *(5.6)* | 17/40 | *(42.5)* |
| Exp. 2, LBA4404 | | 3/632 | *(0.5)* | 1/3 | *(33.3)* |
| | AGL-0 | 10/420 | *(2.4)* | 8/10 | *(80.0)* |
| Exp. 3, LBA4404 | | 0/440 | *(0.0)* | 0/0 | *(0.0)* |
| | AGL-0 | 31/651 | *(4.8)* | 10/31 | *(32.3)* |
| Exp. 4, LBA4404 | | 2/240 | *(0.8)* | 2/2 | *(100)* |
| | AGL-0 | 9/688 | *(1.3)* | 2/9 | *(22.2)* |
| Exp. 5, AGL-0 | | 128/2370 | *(5.4)* | 60/125 | *(48.0)* |
| Total number LBA4404-generated shoots | | 5/2,424 | *(0.2)* | 3/5 | *(60.0)* |
| Total number AGL-0-generated shoots | | | *(4.6)* | 97/215 | *(45.1)* |
| | | 228/5,017 | | | |

| | | | | | |
|---|---|---|---|---|---|
| ^{a}The number of PCR-positive shoots obtained from the total number of shoots tested (%) ^{b}The number of amylose-free transformants obtained from the total number of transformants tested (%) | | | | | |

### Primer Sequences

Sequence identity no. 1 (NPT1):
   5'-TCC ACC TTA TCG GCA ATG AA-3'
Sequence identity no. 2 (NPT2):
   5'-CGG CAG TGA GAG CAG AGA TA-3'
Sequence identity no. 3 (NPT3):
   5'-TCG GCT ATG ACT GGG CAC AAC AGA-3'
Sequence identity no. 4 (NPT4):
   5'-AAG AAG GCG ATA GAA GGC GAT GCG-3'
Sequence identity no. 5 (trfA1):
   5'-TTC TCC TCG TGC TCG TAA AC-3'
Sequence identity no. 6 (trfA2):
   5'-GGT CGC TGG TAT TCG TGC-3'
Sequence identity no. 7 (insB1):
   5'-GCG CTA TCT CTG CTC TCA CT-3'
Sequence identity no. 8 (insB2):
   5'-AAC GGC CTC ACC CCA AAA A-3'
Sequence identity no. 9 (BINMCS):
   5'-GCA CCC CAG GCT TTA CAC TT-3'
Sequence identity no. 10 (GBSS-0):
   5'-TAC CGC TAC CAC TTG ACA TTC-3'
Sequence no. 11:
   5' AGCT GCATATGAAGCTTTCTAGATCTGAATTCCATATGT 3'
   3' CGTATACTTCGAAAGATCTAGACTTAAGGTATACATTAA 5'
Sequence no. 12:
   Primer 5CASGBF1: 5'-TAG AAT TCA CCA GCG GAA CCT ATT TT-3'
Sequence no. 13:
   Primer 5CASGBF2: 5'-ATG AAT TCG GAC CCA AAC TAT CAC TC-3'
Sequence no. 14:
   Primer 5CASGBR1: 5'-TGT CTA GAA TGG AAG CAG AGC AGT GT-3'
Sequence no. 15:
   Primer MCASGBF1: 5'-CAG AAT TCA AGC CAT TTA CCA ACC TA-3'
Sequence no. 16:
   Primer MCASGBF2:5'-ATG AAT TCT ATG AGA AGC CCG TGA AG-3'
Sequence no. 17:
   Primer MCASGBR1:5'-CAT CTA GAG AAC CAG CAT AAA GTC AG-3'
Sequence no. 18:
   Primer 3CASGBF1: 5'-TAG AAT TCG GCT TCA TTG GTA GAT TA-3'
Sequence no. 19:
   Primer 3CASGBF2: 5'-TAG AAT TCA TTG AGC ATC TGG AGG TT-3'
Sequence no. 20:
   Primer 3CASGBR1: 5'-TAT CTA GAC CAT TCA CCC TTC ACA AA-3'

## Claims

1. A method for obtaining a marker-free, uniform transgenic plant, consisting essentially of the steps of:
- transforming a plant cell with a recombinant nucleic acid comprising a T-DNA construct wherein said T-DNA is provided with a foreign nucleic acid that is free of a nucleic acid encoding a selective marker
- regeneration of said cell under no selective pressure
- testing said cell or progeny thereof for the presence or absence of at least a functional part of said foreign nucleic acid and identify transformed plant cells or progeny thereof
- growing a plant from said identified cell or progeny thereof
- testing the obtained plant for uniformness and selecting a uniform plant.

2. A method according to claim 1, wherein said nucleic acid that comprises a foreign nucleic acid that is free of nucleic acid encoding a selective marker is delivered to said plant cell by an Agrobacterium strain.

3. A method according to claim 3, wherein said Agrobacterium strain is a virulent Agrobacterium strain.

4. A method according to claim 2 or 3 wherein said Agrobacterium strain carries a DNA region originating from the virulence region of the Ti plasmid pTiBo542.

5. A method according to any one of claims 2 to 4, wherein said Agrobacterium strain is selected from EHA101, AGL-0, AGL-1.

6. A method according to any one of claims 1 to 5 wherein said foreign nucleic acid allows for regulation of the expression of a target gene in the genome of said plant cell.

7. A method according to claim 6 wherein said regulation comprises downregulation.

8. A method according to claim 6 or 7 wherein said foreign nucleic acid includes an inverted repeat of at least part of a polynucleotide region of said target gene.

9. A method according to any one of claims 6 to 8, wherein said target gene encodes a granule-bound starch synthase (GBSSI) enzyme.

10. A method according to any one of claims 1 to 9, wherein said foreign nucleic acid allows for expression of a heterologous polypeptide in the genome of said plant cell.

11. A method according to claim 10, wherein said heterologous polypeptide comprises an enzyme.

12. A method according to claim 11, wherein said enzyme comprises a , preferably feedback-insensitive, dihydrodipicolinate synthase (DHPS)

13. A method according to any one of claims 1 to 12, wherein said testing for the presence or absence of at least a functional part of said foreign nucleic acid is performed by nucleic acid techniques, ELISA, bioassay or chemical analytical methods.

14. A method according to claim 13, wherein said testing is performed by PCR.

15. A plant, or part thereof, obtained by the method of any of claims 1-14.

16. A tuberous plant, or part thereof, according to claim 15.

17. A plant, or part thereof, according to claim 16 which is selected from potato or cassava plants.
